(19) Europäisches Patentamt
European Patent Office
Office européen des brevets

(11) EP 0 726 903 B1

(12) EUROPEAN PATENT SPECIFICATION

(45) Date of publication and mention of the grant of the patent:
**16.07.1997 Bulletin 1997/29**

(21) Application number: **94932023.8**

(22) Date of filing: **03.11.1994**

(51) Int Cl.[6]: **C07F 9/30**, A61K 31/66, C07F 9/60, C07F 9/58, C07C 233/47, C07C 255/60, C07C 323/52, C07C 327/22, C07D 209/20, C07C 259/06

(86) International application number:
**PCT/US94/12214**

(87) International publication number:
**WO 95/12603 (11.05.1995 Gazette 1995/20)**

# (54) MATRIX METALLOPROTEASE INHIBITORS

MATRIX METALLPROTEASE INHIBITOREN

INHIBITEURS DE METALLOPROTEASES MATRICIELLES

(84) Designated Contracting States:
**AT BE CH DE DK ES FR GB GR IE IT LI LU MC NL PT SE**

(30) Priority: **04.11.1993 US 147811**

(43) Date of publication of application:
**21.08.1996 Bulletin 1996/34**

(73) Proprietor: **SYNTEX (U.S.A.) INC.**
**Palo Alto, CA 94304 (US)**

(72) Inventors:
- **CASTELHANO, Arlindo, L.**
  **Pleasanton, CA 94566 (US)**
- **YUAN, Zhengyu**
  **Fremont, CA 94536 (US)**
- **HORNE, Stephen**
  **Burlington, Ontario L7R 1R5 (CA)**
- **LIAK, Teng, J.**
  **Mississauga, Ontario L5M 4R6 (CA)**

(74) Representative: **Witte, Hubert et al**
**F.Hoffmann-La Roche AG**
**Patent Department (PLP),**
**124 Grenzacherstrasse**
**4070 Basel (CH)**

(56) References cited:
**WO-A-92/09563** **WO-A-93/14112**
**WO-A-94/07481** **US-A- 5 183 900**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 726 903 B1

## Description

### Field of the Invention

The present invention is directed to compounds and their pharmaceutically acceptable salts, which inhibit matrix metalloproteases, particularly stromelysin and matrilysin, and are therefore useful in the treatment of mammals having disease-states alleviated by the inhibition of such matrix metalloproteases.

### BACKGROUND OF THE INVENTION

Matrix metalloproteases are a family of proteases responsible for degradation and remodeling of connective tissues. Members of this family of enzymes share a number of properties, including zinc and calcium dependence, secretion as zymogens, and 40-50% amino acid sequence homology. The matrix metalloprotease family includes interstitial collagenases, derived from fibroblasts/macrophages and neutrophils, which catalyze the initial and rate-limiting cleavage of native collagen types I, II, III and X.

Collagen, the major structural protein of mammals, is an essential component of the matrix of many tissues, for example, cartilage, bone, tendon and skin. Interstitial collagenases are very specific matrix metalloproteases which cleave collagen to give two fragments which spontaneously denature at physiological temperatures and therefore become susceptible to cleavage by less specific enzymes. As cleavage by the collagenase results in the loss of structural integrity of the target tissue, it is essentially an irreversible process and therefore a good target for therapeutic intervention.

In addition to interstitial collagenases, the matrix metalloprotease family of enzymes include two distinct, but highly related, gelatinases: a 72-kD enzyme secreted by fibroblasts and a 92-kD enzyme release by mononuclear phagocytes. These gelatinases are capable of degrading gelatins (denatured collagens), native collagen types IV and V, fibronectin and insoluble elastin.

The matrix metalloprotease family also includes stromelysins 1 and 2, which are capable of cleaving a broad range of matrix substrates, including laminin, fibronectin, proteoglycans and collagen types IV and IX in their non-helical domains.

Matrilysin (putative metalloprotease or PUMP) is a recently described member of the matrix metalloprotease family. Matrilysin is capable of degrading a wide range of matrix substrates including proteoglycans, gelatins, fibronectin, elastin, and laminin. Its expression has been documented in mononuclear phagocytes, rate uterine explants and sporadically in tumors.

Inhibitors of matrix metalloproteases are considered to provide useful treatments for arthritic diseases, bone resorption disease (such as osteoporosis), the enhanced collagen destruction associated with diabetes, periodontal disease, corneal ulceration, ulceration of the skin, and tumor metastasis. For example, the design and potential use of collagenase inhibitors is described in *J. Enzyme Inhibition* (1987), Vol. 2, pp. 1-22, and in *Drug News* & *Prospectives* (1990), Vol. 3, No. 8, pp. 453-458. Matrix metalloprotease inhibitors are also the subject of various patents and patent applications, for example, U.S. Patent Nos. 5,189,178 (Galardy) and 5,183,900 (Galardy), European Published Patent Applications 0 438 223 (Beecham) and 0 276 436 (F. Hoffmann-La Roche), Patent Cooperation Treaty International Applications 92/21360 (Merck), 92/06966 (Beecham) and 92/09563 (Glycomed).

### SUMMARY OF THE INVENTION

The invention provides new compounds which are useful as inhibitors of matrix metalloproteases, particularly stromelysin and matrilysin, and which are effective in treating disease-states characterized by excessive activity of matrix metalloproteases.

Accordingly, one aspect of the invention is directed to compounds of formula (I):

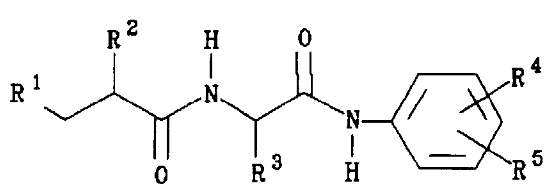

(I)

wherein

$R^1$ is mercapto, acetylthio, carboxy, hydroxycarbamoyl, alkoxycarbonyl, aryloxycarbonyl, aralkoxycarbonyl, benzyloxyaminocarbonyl or

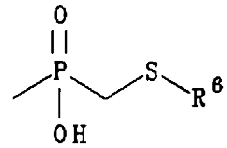

(where $R^6$ is optionally substituted aryl, wherein the aryl group is quinol-2-yl, naphth-1-yl, naphth-2-yl, pyridyl or phenyl);

$R^2$ is alkyl, aralkyl or cycloalkylalkyl;

$R^3$ is cycloalkyl, alkyl (optionally substituted by cycloalkyl, hydroxy, mercapto, alkylthio, aralkoxy, carboxy, amino, alkylamino, guanidino, carbamoyl, pyridyl or indolyl), or aralkyl (optionally substituted by hydroxy, carboxy, alkyl or alkoxy);

$R^4$ is nitro, amino, cyano, hydroxy, alkoxy, carboxy, alkoxycarbonyl, alkylsulfonyl, haloalkyl, alkoxycarbonylalkyl, tetrazolyl, carbamoyl (optionally substituted by alkyl or dialkylaminoalkyl), or aminosulfonyl (optionally substituted by alkyl); and

$R^5$ is hydrogen, halo or hydroxy; as a single stereoisomer or as a mixture thereof; or a pharmaceutically acceptable salt thereof.

Another aspect of the invention is directed to methods of inhibiting matrix metalloprotease activity in a mammal, which methods comprise administering to the mammal in need thereof a therapeutically effective amount of a compound of formula (I) as defined above, as a single stereoisomer, or as a mixture thereof, or a pharmaceutically acceptable salt thereof.

Another aspect of the invention is directed to a pharmaceutical composition useful in inhibiting matrix metalloprotease activity in a mammal, which composition comprises a therapeutically effective amount of a compound of formula (I) as defined above, as a single stereoisomer or as a mixture thereof; or a pharmaceutically acceptable salt thereof; and a pharmaceutically acceptable excipient.

## DETAILED DESCRIPTION OF THE INVENTION

### Definitions

As used in the specification and appended claims, unless specified to the contrary, the following terms have the meaning indicated:

"BOC" refers to *t*-butoxycarbonyl.

"CBZ" refers to benzyloxycarbonyl (carbobenzyloxy).

"DMAP" refers to *N,N*-dimethylaminopyridine.

"DMF" refers to *N,N*-dimethylformamide.

"EDCI" refers to *N*-ethyl-*N'*-(3-dimethylaminopropyl) carbodiimide.

"HOBT" refers to 1-hydroxybenzotriazole.

"Hydroxy" refers to the radical -OH.

"Amino" refers to the radical $-NH_2$.

"Acetylthio" refers to the radical $-SC(O)CH_3$.

"Halo" refers to bromo, chloro or fluoro.

"Carbamoyl" refers to the radical $-C(O)NH_2$.

"Carboxy" refers to the radical -C(O)OH.

"Hydroxyamino" refers to the radical -NHOH.

"Hydroxycarbamoyl" refers to the radical -C(O)NHOH.

"Mercapto" refers to the radical -SH.

"Benzyloxycarbamoyl" refers to $-C(O)N(H)OCH_2C_6H_5$.

"Alkyl" refers to a straight or branched chain monovalent radical consisting solely of carbon and hydrogen, containing no unsaturation and having from one to four carbon atoms, *e.g.,* methyl, ethyl, *n*-propyl, 2-methylpropyl (*iso*-butyl), 1-methylethyl (*iso*-propyl), *n*-butyl, and 1,1-dimethylethyl (*t*-butyl), and the like.

"Alkylamino" refers to a radical of the formula $-NHR_a$ where $R_a$ is alkyl as defined above, *e.g.,* methylamino, ethylamino, *iso*-propylamino, *n*-butylamino, and the like.

"Haloalkyl" refers to a radical of the formula $-R_aR_d$ where $R_a$ is alkyl as defined above substituted by one or more halo groups ($R_d$) as defined above, *e.g.,* 2-chloroethyl, 2-bromoethyl, trifluoromethyl, and the like.

"Dialkylaminoalkyl" refers to a radical of the formula $-R_aN(R_a)_2$ where each $R_a$ is independently an alkyl radical as defined above, *e.g.,* dimethylaminoethyl, diethylamino-*n*-propyl, dimethylamino-*n*-propyl, and the like.

"Aminosulfonyl" refers to $-S(O)_2NH_2$.

"Alkylsulfonyl" refers to a radical of the formula $-S(O)_2R_a$ where $R_a$ is alkyl as defined above, *e.g.,* methylsulfonyl, ethylsulfonyl, *iso*propylsulfonyl, and the like.

"Alkylthio" refers to a radical of the formula $-SR_a$ where $R_a$ is alkyl as defined above, *e.g.,* methylthio, ethylthio, *iso*-propylthio, *n*-butylthio, and the like.

"Alkoxy" refers to a radical of the formula $-OR_a$ wherein $R_a$ is alkyl as defined above, *e.g.*, methoxy, ethoxy, *n*-propoxy, 1-methylethoxy, *n*-butoxy, *t*-butoxy, and the like.

"Alkoxycarbonylalkyl" refers to a radical of the formula $-R_aC(O)R_b$ where $R_a$ is alkyl as defined above and $R_b$ is alkoxy as defined above, *e.g.,* methoxycarbonylethyl, ethoxycarbonylethyl, methoxycarbonyl-*iso*-propyl, and the like.

"Aryl" refers to a quinol-2-yl, naphth-1-yl, naphth-2-yl or phenyl radical.

"Aryloxy" refers to a radical of the formula $-OR_b$ wherein $R_b$ is aryl as defined above, *e.g.,* phenoxy, quinol-2-yloxy, naphth-1-yloxy, or naphth-2-yloxy.

"Aralkyl" refers to a radical of the formula $-R_aR_b$ wherein $R_a$ is alkyl as defined above and $R_b$ is aryl as defined above, *e.g.,* benzyl, phenylethylene, 3-phenylpropyl, 2-quinol-2-ylethyl, and the like.

"Aralkoxy" refers to a radical of the formula $-OR_aR_b$ wherein $R_a$ is alkyl as defined above and $R_b$ is aryl as defined above, *e.g.,* benzyloxy, 2-quinol-2-ylethoxy, 3-naphth-2-ylpropoxy, and the like.

"Alkoxycarbonyl" refers to a radical of the formula $-C(O)R_b$ wherein $R_b$ is alkoxy as defined above, *e.g.,* methoxycarbonyl, ethoxycarbonyl, *t*-butoxycarbonyl, and the like.

"Aralkoxycarbonyl" refers to a radical of the formula $-C(O)R_c$ wherein $R_c$ is aralkoxy as defined above, *e.g.,* benzyloxycarbonyl, 2-quinol-2-ylethoxycarbonyl, and the like.

"Cycloalkyl" refers to a monovalent ring radical consisting solely of carbon and hydrogen atoms, containing no unsaturation and having from five to seven carbon atoms, *e.g.,* cyclopentyl, cyclohexyl and cycloheptyl.

"Cycloalkylalkyl" refers to a radical of the formula $-R_eR_a$ where $R_a$ is alkyl as defined above and $R_e$ is cycloalkyl as defined above, *e.g.,* cyclohexylmethyl, cyclohexylethyl, cyclopentylmethyl, cyclopentylmethyl, and the like.

"Phosphinoyl" refers to a radical of the formula $H_2P(O)$-, in which the 2 H atoms may be replaced by other groups.

"Optional" or "optionally" means that the subsequently described event of circumstances may or may not occur, and that the description includes instances where said event or circumstance occurs and instances in which it does not. For example, "optionally substituted quinol-2-yl" means that the quinol-2-yl radical may or may not be substituted and that the description includes both substituted quinol-2-yl radicals and quinol-2-yl radicals having no substitution.

"Optionally substituted aryl" refers to a quinol-2-yl, naphth-1-yl, naphth-2-yl, pyridyl or phenyl radical optionally substituted by one or more substituents selected from the group consisting of halo, alkyl, alkoxy, hydroxy, and nitro, *e.g.*, 6-nitroquinol-2-yl, 6-fluoroquinol-2-yl, 6-hydroxyquinol-2-yl, 6-methoxyquinol-2-yl, 6-nitronaphth-1-yl, 6-chloronaphth-1-yl, 6-hydroxynaphth-1-yl, 6-methoxynaphth-1-yl, 6-nitronaphth-2-yl, 6-chloronaphth-2-yl, 6-hydroxynaphth-2-yl, 6-methoxynaphth-2-yl, 6-nitrophenyl, 6-chlorophenyl, 6-hydroxyphenyl, 6-methoxyphenyl, and the like.

"Amino-protecting group" as used herein refers to those organic groups intended to protect nitrogen atoms against undesirable reactions during synthetic procedures, and includes, but is not limited to, benzyl, acyl, acetyl, benzyloxycarbonyl (carbobenzyloxy), p-methoxybenzyloxycarbonyl, p-nitrobenzyloxycarbonyl, *t*-butoxycarbonyl, and the like.

"Pharmaceutically acceptable salt" refers to those salts which retain the biological effectiveness and properties of the free bases or free acids and which are not biologically or otherwise undesirable. If the compound exists as a free acid, the desired salt may be prepared by methods known to those of ordinary skill in the art, such as treatment of the compound with an inorganic acids such as hydrochloric acid, hydrobromic acid, sulfuric acid, nitric acid, phosphoric acid and the like; or with an organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like. If the compound exists as a free base, the desired salt may also be prepared by methods known to those of ordinary skill in the art, such as the treatment of the compound with an inorganic base or an organic base. Salts derived from inorganic bases include, but are not limited to, the sodium, potassium, lithium, ammonium, calcium, magnesium, iron, zinc, copper, manganese, aluminum salts and the like. Salts derived from organic bases include, but are not limited to, salts of primary, secondary, and tertiary amines, substituted amines including naturally occurring substituted amines, cyclic amines and basic ion exchange resins, such as isopropylamine, trimethylamine, diethylamine, triethylamine, tripropylamine, ethanolamine, 2-dimethylaminoethanol, 2-diethylaminoethanol, trimethamine, dicyclohexylamine, lysine, arginine, histidine, caffeine, procaine, hydrabamine, choline, betaine, ethylenediamine, glucosamine, methylglucamine, theobromine, purines, piperazine, piperidine, *N*-ethylpiperidine, polyamine resins and the like.

"Mammal" includes humans and all domestic and wild animals, including, without limitation, cattle, horses, swine,

sheep, goats, dogs, cats, and the like.

"Therapeutically effective amount" refers to that amount of a compound of formula (I) which, when administered to a mammal in need thereof, is sufficient to effect treatment, as defined below, for disease-states alleviated by the inhibition of matrix metalloprotease activity, particularly stromelysin and matrilysin activity. The amount of a compound of formula (I) which constitutes a "therapeutically effective amount" will vary depending on the compound, the disease-state and its severity, and the mammal to be treated, but can be determined routinely by one of ordinary skill in the art having regard to his own knowledge and to this disclosure.

"Treating" or "treatment" as used herein cover the treatment of a disease-state in a mammal, particularly in a human, which disease-state is alleviated by the inhibition of matrix metalloprotease activity, particularly stromelysin and matrilysin activity, and the like; and include:

(i) preventing the disease-state from occurring in a mammal, in particular, when such mammal is predisposed to the disease-state but has not yet been diagnosed as having it;
(ii) inhibiting the disease-state, i.e., arresting its development; or
(iii) relieving the disease-state, i.e., causing regression of the disease-state.

"Stereoisomers" refers to compounds having identical molecular formulae and nature or sequence of bonding but differing in the arrangement of their atoms in space.

The nomenclature used herein is basically a modified form of I.U.P.A.C. nomenclature wherein the compounds of the invention are named as peptide derivatives. The compounds of formula (I), or their pharmaceutically acceptable salts, have at least two asymmetric carbon atoms in their structure, and may therefore exist as single stereoisomers, racemates, and as mixtures of enantiomers and diastereomers. All such single stereoisomers, racemates and mixtures thereof are intended to be within the scope of this invention.

When naming the single stereoisomers of compounds of formula (I) an absolute descriptor, *R* or *S*, may be assigned to the chiral carbon atoms therein according to the "Sequence Rule" procedure of Cahn, Ingold and Prelog.

In addition, those sections of compounds of formula (I) which include $R^3$, together with the adjacent nitrogen atom and carbonyl group, may define an $\alpha$-amino acid residue and are named as such.

For example, the following compound of formula (I):

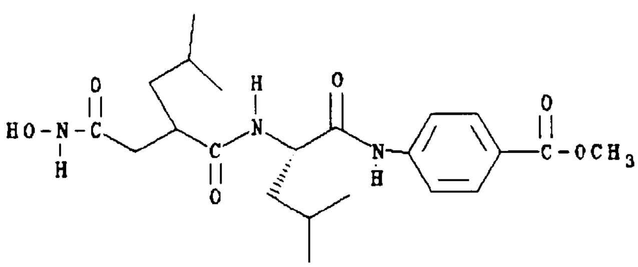

wherein $R^1$ is hydroxycarbamoyl; $R^2$ is 2-methylpropyl; $R^3$ is 2-methylpropyl; $R^4$ is methoxycarbonyl; and $R^5$ is hydrogen; is named herein as *N*- (4-methyl-2-(*N"*-hydroxycarbamoyl)methyl-pentanoyl)-L-leucine-*N'*-(4-methoxycarbonylphenyl)carboxamide.

**Utility and Administration**

**A. Utility**

The compounds of formula (I) are useful in inhibiting mammalian matrix metalloproteases, particularly stromelysin and matrilysin, thereby preventing the degradation of collagen within the mammal. The compounds are therefore useful in treating disease-states which are associated with increased activity of matrix metalloproteases, particularly increased activity of stromelysin and matrilysin, such as arthritis and osteoarthritis, tumor metastasis, periodontal disease and corneal ulcerations. See, *e.g., Arthritis and Rheumatism* (1993), Vol. 36, No. 2, pp. 181-189; *Arthritis and Rheumatism* (1991), Vol. 34, No. 9, pp. 1073-1075; *Seminars in Arthritis and Rheumatism* (1990), Vol. 19, No. 4, Supplement 1 (February), pp. 16-20; *Drugs of the Future* (1990), Vol. 15, No. 5, pp. 495-508; and *J. Enzyme Inhibition* (1987), Vol. 2, pp. 1-22.

**B. Testing**

The ability of the compounds of formula (I) to inhibit matrix metalloprotease activity, particularly stromelysin or matrilysin activity may be demonstrated by a variety of *in vitro* and *in vivo* assays known to those of ordinary skill in the art, such as the assay described in *Anal. Biochem.* (1985), Vol. 147, p. 437, or modifications thereof.

**C. General Administration**

Administration of the compounds of formula (I), or their pharmaceutically acceptable salts, in pure form or in an appropriate pharmaceutical composition, can be carried out via any of the accepted modes of administration or agents for serving similar utilities. Thus, administration can be, for example, orally, nasally, parenterally, topically, transdermally, or rectally, in the form of solid, semi-solid, lyophilized powder, or liquid dosage forms, such as tablets, suppositories, pills, soft elastic and hard gelatin capsules, powders, solutions, suspensions, or aerosols, or the like, preferably in unit dosage forms suitable for simple administration of precise dosages. The compositions will include a conventional pharmaceutical carrier or excipient and a compound of formula (I) as the/an active agent, and, in addition, may include other medicinal or pharmaceutical agents, carriers, adjuvants, *etc.*

Generally, depending on the intended mode of administration, the pharmaceutically acceptable compositions will contain about 1% to about 99% by weight of a compound(s) of formula (I), or a pharmaceutically acceptable salt thereof, and 99% to 1% by weight of a suitable pharmaceutical excipient. Preferably, the composition will be about 5% to 75% by weight of a compound(s) of formula (I), or a pharmaceutically acceptable salt thereof, with the rest being suitable pharmaceutical excipients.

The preferred route of administration is oral, using a convenient daily dosage regimen which can be adjusted according to the degree of severity of the disease-state to be treated. For such oral administration, a pharmaceutically acceptable composition containing a compound(s) of formula (I), or a pharmaceutically acceptable salt thereof, is formed by the incorporation of any of the normally employed excipients, such as, for example, pharmaceutical grades of mannitol, lactose, starch, pre-gelatinized starch, magnesium stearate, sodium saccharine, talcum, cellulose ether derivatives, glucose, gelatin, sucrose, citrate, propyl gallate, and the like. Such compositions take the form of solutions, suspensions, tablets, pills, capsules, powders, sustained release formulations and the like.

Preferably such compositions will take the form of capsule, caplet or tablet and therefore will also contain a diluent such as lactose, sucrose, dicalcium phosphate, and the like; a disintegrant such as croscarmellose sodium or derivatives thereof; a lubricant such as magnesium stearate and the like; and a binder such as a starch, gum acacia, polyvinylpyrrolidone, gelatin, cellulose ether derivatives, and the like.

The compounds of formula (I), or their pharmaceutically acceptable salts, may also be formulated into a suppository using, for example, about 0.5% to about 50% active ingredient disposed in a carrier that slowly dissolves within the body, *e.g.,* polyoxyethylene glycols and polyethylene glycols (PEG), *e.g.*, PEG 1000 (96%) and PEG 4000 (4%).

Liquid pharmaceutically administrable compositions can, for example, be prepared by dissolving, dispersing, *etc.,* a compound(s) of formula (I) (about 0.5% to about 20%), or a pharmaceutically acceptable salt thereof, and optional pharmaceutical adjuvants in a carrier, such as, for example, water, saline, aqueous dextrose, glycerol, ethanol and the like, to thereby form a solution or suspension.

If desired, a pharmaceutical composition of the invention may also contain minor amounts of auxiliary substances such as wetting or emulsifying agents, Ph buffering agents, antioxidants, and the like, such as, for example, citric acid, sorbitan monolaurate, triethanolamine oleate, butylated hydroxytoluene, *etc.*

Actual methods of preparing such dosage forms are known, or will be apparent, to those skilled in this art; for example, see *Remington's Pharmaceutical Sciences,* 18th Ed., (Mack Publishing Company, Easton, Pennsylvania, 1990). The composition to be administered will, in any event, contain a therapeutically effective amount of a compound of formula (I), or a pharmaceutically acceptable salt thereof, for treatment of a disease-state alleviated by the inhibition of matrix metalloprotease activity in accordance with the teachings of this invention.

The compounds of formula (I), or their pharmaceutically acceptable salts, are administered in a therapeutically effective amount which will vary depending upon a variety of factors including the activity of the specific compound employed, the metabolic stability and length of action of the compound, the age, body weight, general health, sex, diet, mode and time of administration, rate of excretion, drug combination, the severity of the particular disease-state, and the host undergoing therapy. Generally, a therapeutically effective daily dose is from about 0.14 mg to about 14.3 mg/kg of body weight per day of a compound of formula (I), or a pharmaceutically acceptable salt thereof; preferably, from about 0.7 mg to about 10 mg/kg of body weight per day; and most preferably, from about 1.4 mg to about 7.2 mg/kg of body weight per day. For example, for administration to a 70 kg person, the dosage range would be from about 10 mg to about 1.0 gram per day of a compound of formula (I), or a pharmaceutically acceptable salt thereof, preferably from about 50 mg to about 700 mg per day, and most preferably from about 100 mg to about 500 mg per day.

**Preferred Embodiments**

A preferred group of the compounds of formula (I), as described above in the Summary of the Invention, are those compounds wherein $R^1$ is mercapto or acetylthio.

Within this group, a preferred subgroup of compounds are those compounds wherein $R^2$ is alkyl, aralkyl, cycloalkylalkyl; $R^3$ is cycloalkyl or alkyl (optionally substituted by cycloalkyl, hydroxy, aralkoxy, alkylthio, pyridyl or indolyl); $R^4$ is cyano, carboxy, hydroxy, alkoxy, alkoxycarbonyl, alkoxycarbonylalkyl, carbamoyl (optionally substituted by aralkylaminoalkyl), or aminosulfonyl (optionally substituted by alkyl); and $R^5$ is hydrogen.

Within this subgroup, a preferred class of compounds are those compounds wherein $R^2$ is alkyl; $R^3$ is cyclohexyl, alkyl (optionally substituted by cyclohexyl, hydroxy, benzyloxy, methylthio, pyridyl or indolyl); and $R^4$ is carboxy, alkoxycarbonyl and aminosulfonyl.

Within this class of compounds, compounds wherein $R^2$ is 2-methylpropyl are preferred. Particularly preferred are those compounds wherein $R^3$ is 2-methylpropyl.

Another preferred group of compounds of formula (I), as described above in the Summary of the Invention, are those compounds wherein $R^1$ is carboxy.

Within this group, a preferred subgroup of compounds are those compounds wherein $R^2$ is alkyl, aralkyl, cycloalkylalkyl; $R^3$ is cycloalkyl or alkyl (optionally substituted by cycloalkyl, hydroxy, aralkoxy, alkylthio, pyridyl or indolyl); $R^4$ is cyano, hydroxy, alkoxy, carboxy, alkoxycarbonyl, alkoxycarbonylalkyl, carbamoyl (optionally substituted by aralkylaminoalkyl), or aminosulfonyl (optionally substituted by alkyl); and $R^5$ is hydrogen.

Within this subgroup, a preferred class of compounds are those compounds wherein $R^2$ is alkyl; $R^3$ is cyclohexyl, alkyl (optionally substituted by cyclohexyl, hydroxy, benzyloxy, methylthio, pyridyl or indolyl); and $R^4$ is carboxy, alkoxycarbonyl and aminosulfonyl.

Within this class of compounds, preferred compounds are those compounds wherein $R^2$ is 2-methylpropyl. Particularly preferred are those compounds wherein $R^3$ is cyclohexyl, 2-methylpropyl, pyrid-3-ylmethyl, 1-benzyloxyethyl, 1-methylpropyl, 1,1-dimethylethyl, 1-hydroxyethyl, and indol-2-ylmethyl; and $R^4$ is methoxycarbonyl.

Another preferred group of the compounds of formula (I), as described above in the Summary of the Invention, are those compounds wherein $R^1$ is hydroxycarbamoyl.

Within this group, a preferred subgroup of compounds are those compounds wherein $R^2$ is alkyl, aralkyl, cycloalkylalkyl; $R^3$ is cycloalkyl or alkyl (optionally substituted by cycloalkyl, hydroxy, aralkoxy, alkylthio, pyridyl or indolyl); $R^4$ is cyano, hydroxy, alkoxy, carboxy, alkoxycarbonyl, alkoxycarbonylalkyl, carbamoyl (optionally substituted by aralkylaminoalkyl), or aminosulfonyl (optionally substituted by alkyl); and $R^5$ is hydrogen.

Within this subgroup, a preferred class of compounds are those compounds wherein $R^2$ is alkyl; $R^3$ is cyclohexyl, alkyl (optionally substituted by cyclohexyl, hydroxy, benzyloxy, methylthio, pyridyl or indolyl); and $R^4$ is carboxy, alkoxycarbonyl and aminosulfonyl.

Within this class, preferred compounds are those compounds wherein $R^2$ is 2-methylpropyl. Particularly preferred are those compounds wherein $R^3$ is cyclohexyl, 2-methylpropyl, pyrid-3-ylmethyl, 1-benzyloxyethyl, 1-methylpropyl, 1,1-dimethylethyl, 1-hydroxyethyl, and indol-2-ylmethyl.

Presently, the most preferred compounds of formula (I) are the following:

*N*-(4-methyl-2-(*N''*-hydroxycarbamoyl)methylpentanoyl)-L-tryptophan-*N'*-(4-carboxyphenyl)carboxamide;
*N*-(4-methyl-2-(*N''*-hydroxycarbamoyl)methylpentanoyl)-L-leucine-*N'*-(4-methoxycarbonylphenyl)carboxamide;
*N*- (4-methyl-2-(*N''*-hydroxycarbamoyl)methylpentanoyl)-L-leucine-*N'*-(4-carboxyphenyl)carboxamide;
*N*-(4-methyl-2-mercaptomethylpentanoyl)-L-leucine-*N'*-(4-methoxycarbonylphenyl)carboxamide;
*N*-(4-methyl-2-acetylthiomethylpentanoyl)-L-leucine-*N'*-(4-methoxycarbonylphenyl)carboxamide;
*N*-(4-methyl-2-carboxymethylpentanoyl)-L-leucine-*N'*-(4-methoxycarbonylphenyl)carboxamide;
*N*-(4-methyl-2-(*N''*-hydroxycarbaanoyl)methylpentanoyl)-L-cyclohexylglycine-*N'*-(4-methoxycarbonylphenyl)carboxamide; and
*N*-(4-methyl-2-(*N''*-hydroxycarbamoyl)methylpentanoyl)-L-*t*-leucine-*N'*-(4-methoxycarbonyl-phenyl)carboxamide.

**Preparation of Compounds of Formula (I)**

Compounds of formula (I), as single stereoisomers, or as mixtures thereof, and their pharmaceutically acceptable salts, are peptide derivatives which can be prepared from the constituent α-amino acid derivative. Standard methods for the formation of peptide bonds are further illustrated by M. Bodanszky *et al., The Practice of Peptide Synthesis* (1984), Springer-Verlag; M. Bodanszky, *Principles of Peptide Synthesis* (1984), Springer-Verlag; J.P. Greenstein *et al., Chemistry of the Amino Acids* (1961), Vol. 1-3, John Wiley and Sons Inc.; G.R. Pettit, *Synthetic Peptides* (1970), Vol. 1-2, Van Nostrand Reinhold Company.

Amide couplings used to form the compounds of formula (I) are generally performed by the carbodiimide method

with reagents such as dicyclohexylcarbodiimide or *N'*-ethyl-*N'*-(3-dimethylaminopropyl)-carbodiimide (EDCI) in the presence of 1-hydroxybenzotriazole (HOBT) in an inert solvent such as dimethylformamide (DMF). Other methods of forming the amide or peptide bond include, but are not limited to synthetic routes via an acid chloride, acyl azide, mixed anhydride or activated ester such as nitrophenyl ester. Typically, solution phase amide couplings with or without peptide fragments are performed.

The selection of protecting groups for the terminal amino or carboxy groups of compounds used in the preparation of the compounds of formula (I) is dictated in part by the particular amide or peptide coupling conditions, and in part by the amino acid and/or peptide components involved in the coupling. Amino-protecting groups commonly used include those which are well-known in the art, for example, benzyloxycarbonyl (carbobenzyloxy), *p*-methoxybenzyloxycarbonyl, *p*-nitrobenzyloxycarbonyl, *t*-butoxycarbonyl (BOC), and the like. It is preferred to use either BOC or benzyloxycarbonyl (CBZ) as the protecting group for the $\alpha$-amino group because of the relative ease of its removal by mild acids, e.g., by trifluoroacetic acid (TFA) or hydrochloric acid in ethyl acetate; or by catalytic hydrogenation.

The individual stereoisomers of compounds of formula (I) may be separated from each other by methods known to those of ordinary skill in the art, e.g., by selective crystallization or by chromatography, and/or by the methods disclosed herein.

Combinations of substituents and/or variables in compounds of formula (I) and intermediates thereof are permissible only if such combinations result in stable compounds.

**A. Preparation of Compounds of Formula (E)**

Compounds of formula (E) are intermediates used in the preparation of compounds of formula (I), and are prepared as shown in the following Reaction Scheme 1 wherein $R^2$ and $R^6$ are as defined above in the Summary of the Invention and $R^{12}$ is mesyl or tosyl:

**Reaction Scheme 1**

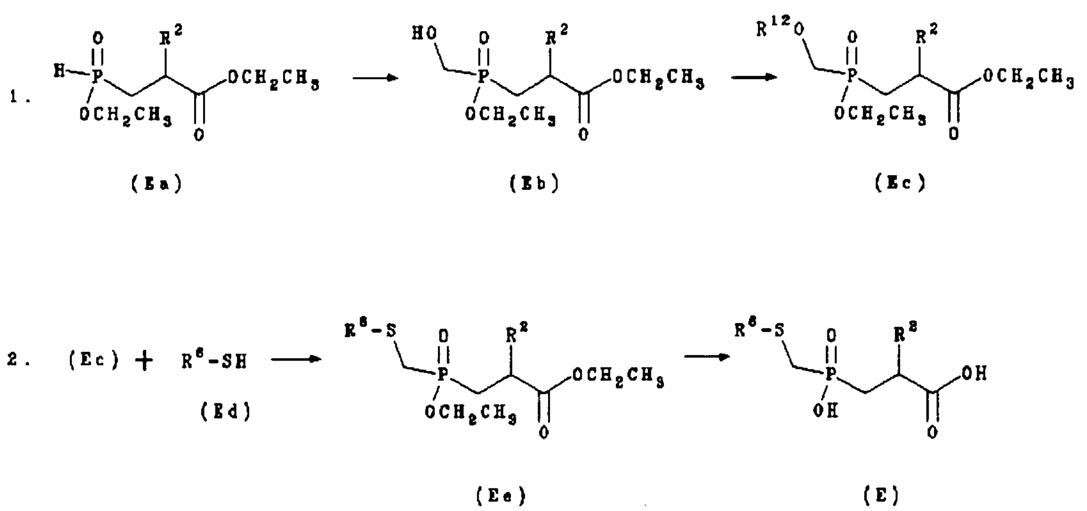

1. (Ea) (Eb) (Ec)

2. (Ec) + (Ed) (Ee) (E)

Compounds of formula (Ea) may be prepared according to methods known to those of ordinary skill in the art (*e. g.*, see European Published Patent Application 0 276 436) or may be prepared according to the method described in Example 1 below. Compounds of formula (Ed) are commercially available or may be prepared according to methods known to those of ordinary skill in the art.

In general, compounds of formula (E) are prepared by first treating a compound of formula (Ea) in an aprotic solvent, preferably tetrahydrofuran and methylene chloride, at 0-15°C, preferably at 0°C, in the presence of a base, preferably diisopropylethylamine and *bis*(trimethylsilyl)acetamide, with paraformaldehyde. The resulting solution is brought to 25-37°C, preferably to 37°C, for 18 hours. The compound of formula (Eb) is then isolated by standard methods, preferably by evaporation of solvent, extraction and filtration.

A compound of formula (Eb) in an aprotic solvent, preferably methylene chloride, is then cooled to -20°C to about 0°C, preferably to about -20°C. The compound of formula (Ec) is then esterified by the standard procedure of treating the alcohol with at least a stoichiometric amount to about a 100% excess of either mesyl chloride or tosyl chloride, for

a period of time, preferably for about 15 minutes, at -20°C, followed by a period of time, preferably for about 3.5 hours, at room temperature. The compound of formula (Ec) is then isolated from the reaction mixture by standard isolation procedures, preferably by extraction, filtration and evaporation.

A compound of formula (Ec) in an aprotic solvent, preferably DMF, is then reacted with a salt of a compound of formula (Ed) (preferably the sodium salt formed from the reaction of the compound of formula (Ed) with sodium hydride in an aprotic solvent, preferably DMF), for about 16-20 hours, preferably for about 18 hours, at temperatures beginning at about 0°C and slowly warming to room temperature. The resulting compound of formula (Ee) is isolated from the reaction mixture by standard isolation techniques, such as by extraction, evaporation, and flash chromatography.

A compound of formula (Ee) is then hydrolyzed under basic conditions, preferably in the presence of sodium hydroxide, to form a compound of formula (E), which is isolated from the reaction mixture by standard isolation techniques.

**B. Preparation of Compounds of Formula (Ia)**

Compounds of formula (Ia) are compounds of formula (I) wherein $R^1$ is

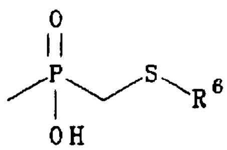

where $R^6$ is optionally substituted aryl, wherein the aryl group is quinol-2-yl, naphth-1-yl, naphth-2-yl, pyridyl or phenyl; $R^2$ is alkyl; $R^3$ is as described above in the Summary of the Invention; $R^4$ is as described above in the Summary of the Invention and $R^5$ is hydrogen. Compounds of formula (Ia) are prepared as described below in Reaction Scheme 2 where $R^2$ is alkyl; $R^3$ is as described above in the Summary of the Invention; $R^4$ is as described above in the Summary of the Invention; $R^5$ is hydrogen; $R^6$ is as described above and BOC is *t*-butoxycarbonyl:

**Reaction Scheme 2**

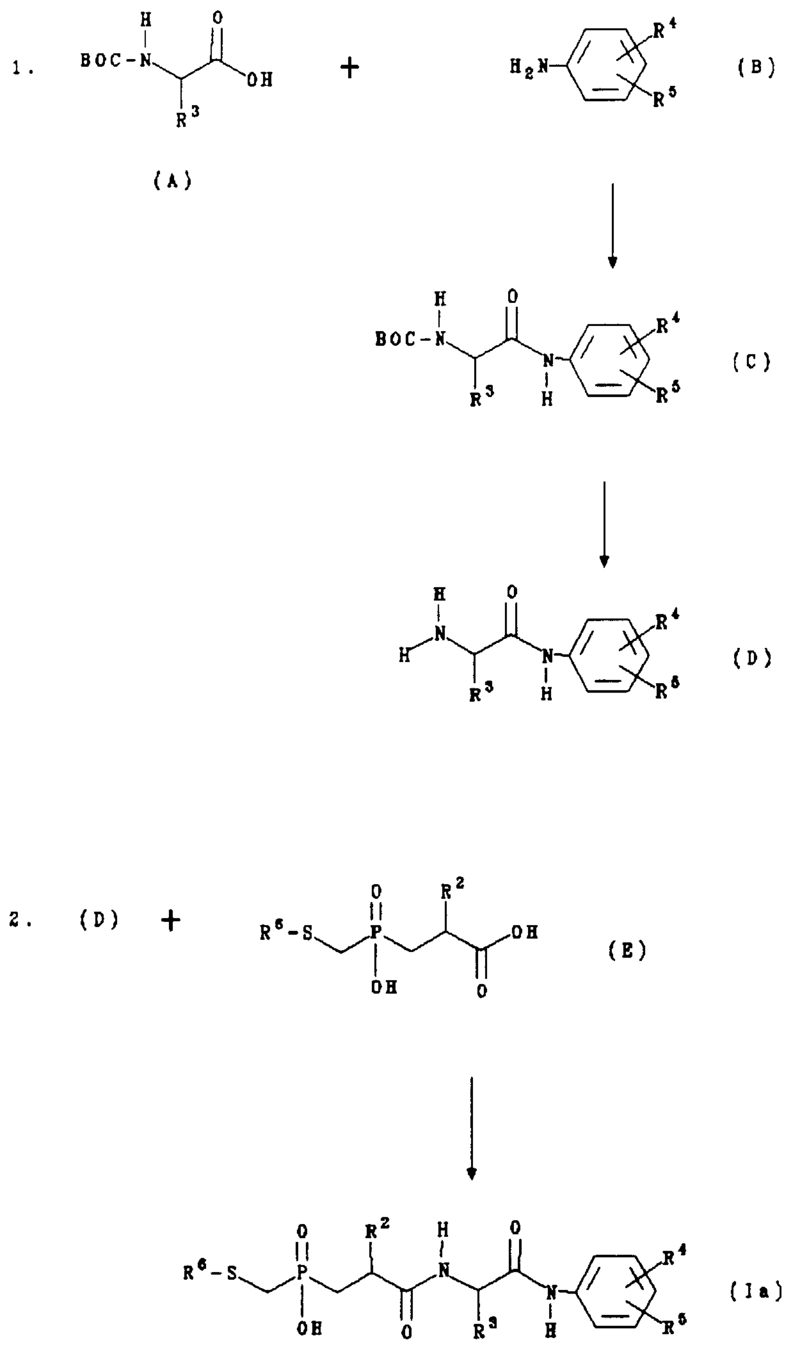

1. (A) (B) (C) (D)

2. (D) + (E) (Ia)

*N*-protected amino acids of formula (A) and compounds of formula (B) are commercially available or may be prepared according to methods known to those of ordinary skill in the art. Compounds of formula (E) are prepared as shown above in Section A.

In general, compounds of formula (Ia) are prepared by first coupling a compound of formula (A) with a compound of formula (B) under standard amide coupling conditions to form a compound of formula (C). For example, to a cold (0-5°C) solution of the compound of formula (A) and an excess molar amount of HOBT in DMF is added an excess molar amount of EDCI. The resulting solution is stirred from about 1 to about 2 hours, preferably for about 1 hour, at 0-5°C, preferably at 0°C. To the cold solution is then added a solution of an equimolar amount of a compound of formula (B) in the presence of a base, preferably DMAP. The resulting mixture is stirred from 12 to 24 hours, preferably for 24

hours, at room temperature, preferably at 25°C. The compound of formula (C) is then isolated from the reaction mixture by isolation techniques standard in the art of peptide chemistry, for example, evaporation, extraction, column chromatography and/or HPLC.

The amino-protecting group of the compound of formula (C) is then removed under mild acidic conditions, preferably in the presence of trifluoroacetic acid, to yield a compound of formula (D).

The compound of formula (D) so formed is then coupled with a compound of formula (E) under standard peptide coupling conditions. For example, to a cold (0-5°C, preferably 0°C) solution of the compound of formula (D) in an inert solvent, preferably THF, is added 1,1'-carbonyldiimidazole. The resulting mixture is stirred from 60 to 90 minutes, preferably for 75 minutes, at 0-5°C, preferably at 0°C, and then reacted with the compound of formula (E) for about 12 to 17 hours, preferably for about 15 hours. The resulting compound of formula (Ia) is then isolated from the reaction mixture by techniques standard is the peptide chemistry, for example, extraction and reverse phase HPLC.

**C. Preparation of Compounds of Formula (F)**

Compounds of formula (F):

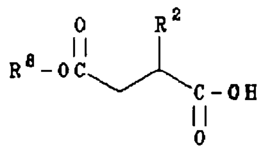

(F)

where $R^2$ is as defined above in the Summary of the Invention and $R^8$ is *t*-butyl, are intermediates in the preparation of compounds of formula (I) and are prepared as shown in following Reaction Scheme 3 where $R^2$ is as described above in the Summary of the Invention and $R^8$ is *t*-butyl:

**Reaction Scheme 3**

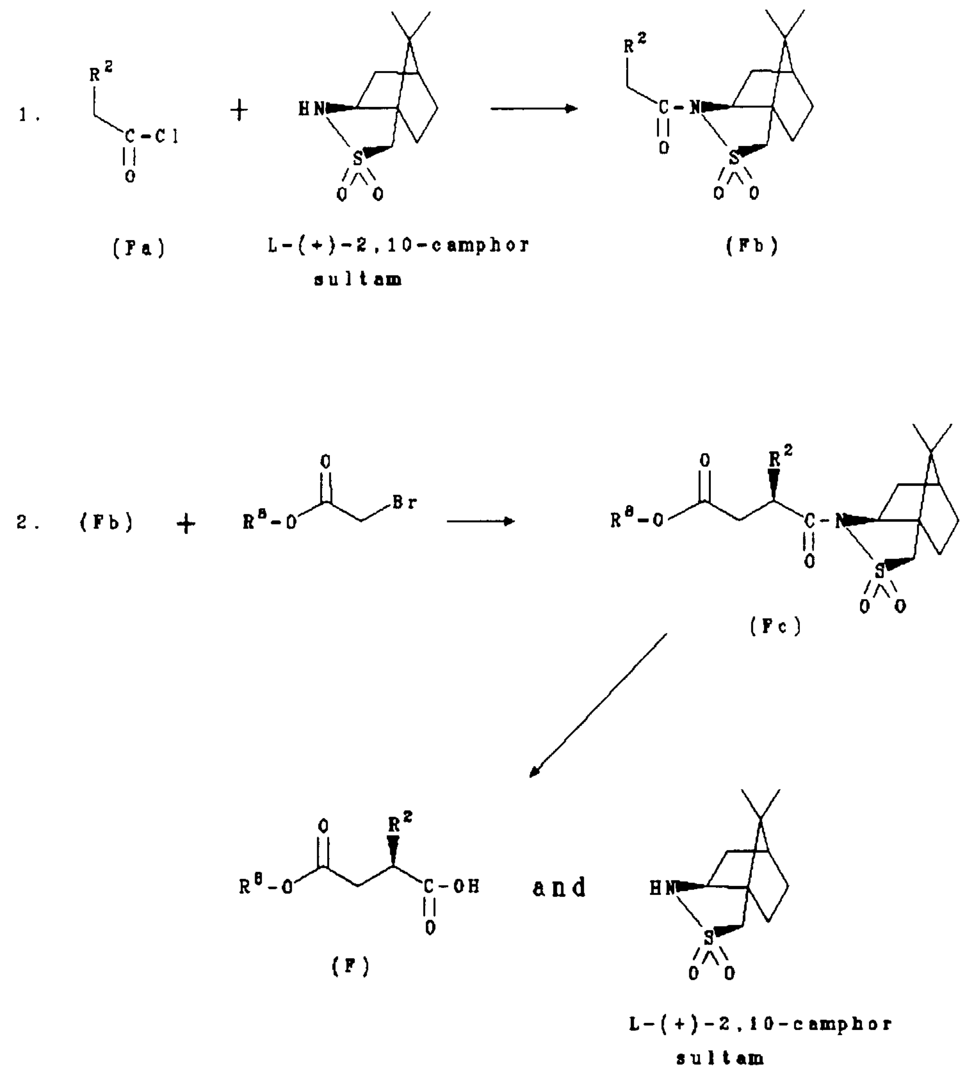

1. (Fa) + L-(+)-2,10-camphor sultam → (Fb)

2. (Fb) + → (Fc) → (F) and L-(+)-2,10-camphor sultam

In a similar manner, but substituting D-(-)-2,10-camphor sultam for L-(+)-2,10-camphor sultam, the corresponding individual stereoisomers in the (*S*) configuration were prepared.

Compounds of formula (Fa) are commercially available or may be prepared according to methods known to those of ordinary skill in the art, for example, by the method described in Example 11 below. L-(+)-2,10-Camphor sultam and D-(-)-2,10-camphor sultam are commercially available, for example, from Aldrich Chemical Company, Milwaukee, Wisconsin, U.S.A.

In general, compounds of formula (F) are prepared by first condensing a compound of formula (Fa) with L-(+)-2,10-camphor sultam to form a compound of formula (Fb). Using NaHMDS to generate the anion for 1 hour, the reaction is quenched with the *t*-butylbromoacetate to form the corresponding ester of formula (Q). The camphor group is then removed under basic conditions to yield an individual stereoisomer of a compound of formula (F) wherein the carbon to which the $R^2$ substituent is attached is in the (*R*) configuration.

**D. Preparation of Compounds of Formulae (Ib), (Ic), (Id) and (Ie)**

Compounds of formula (Ib) are compounds of formula (I) wherein $R^1$ is alkoxycarbonyl or aralkoxycarbonyl; and $R^2$, $R^3$, $R^4$ and $R^5$ are as defined above in the Summary of the Invention.

Compounds of formula (Ic) are compounds of formula (I) wherein $R^1$ is carboxy; and $R^2$, $R^3$, $R^4$ and $R^5$ are as defined above in the Summary of the Invention.

Compounds of formula (Id) are compounds of formula (I) wherein $R^1$ is benzyloxycarbamoyl; and $R^2$, $R^3$, $R^4$ and $R^5$ are as defined above in the Summary of the Invention.

Compounds of formula (Ie) are compounds of formula (I) wherein $R^1$ is hydroxycarbamoyl; and $R^2$, $R^3$, $R^4$ and $R^5$ are as defined above in the Summary of the Invention.

Compounds of formulae (Ib), (Ic), (Id) and (Ie) are prepared as shown in following Reaction Scheme 4 wherein $R^2$, $R^3$, $R^4$, and $R^5$ are as defined above in the Summary of the Invention; and $R^8$ is *t*-butyl.

**Reaction Scheme 4**

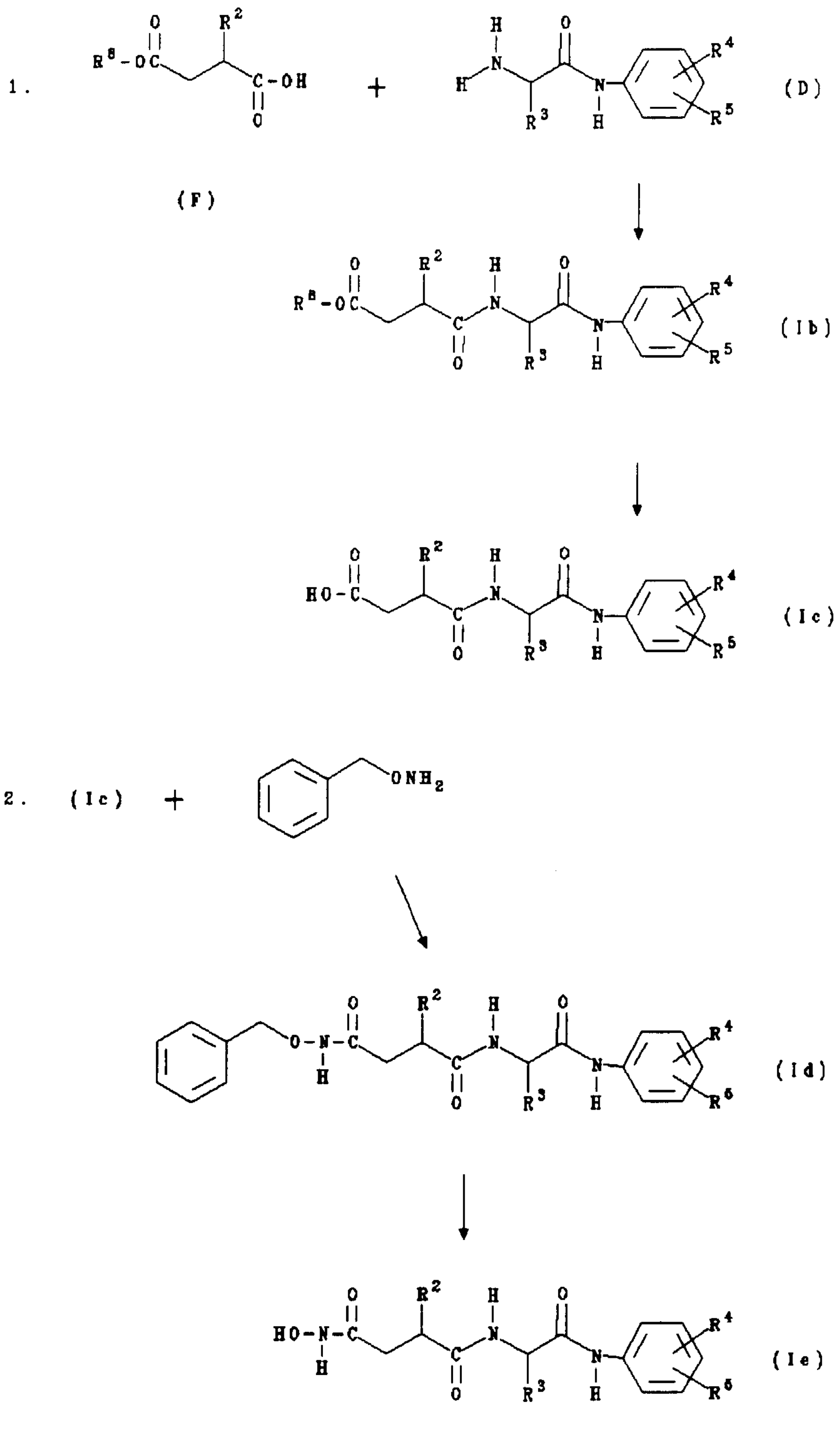

1. (F) + (D) → (Ib) → (Ic)

2. (Ic) + → (Id) → (Ie)

Compounds of formula (F) are prepared as shown above in Section C. Compounds of formula (D) are prepared as shown above in Section B. *O*-Benzylhydroxylamine is commercially available, for example, as the hydrochloride salt from Aldrich Chemical Co.

In general, compounds of formulae (Ib), (Ic), (Id) and (Ie) are prepared by first coupling a compound of formula (F) with a compound of formula (D) under standard amide coupling conditions to form a compound of formula (Ib). For example, to a solution of a compound of formula (F) in an aprotic solvent, preferably DMF, containing a slightly excess molar amount of HOBT, is added an excess molar amount of EDCI. The resulting mixture is stirred for about 1 to about 2 hours, preferably for about 1 hour, at 0-5°C, preferably at 0°C. To the cold solution is then added an equimolar amount of a compound of formula (D) in the presence of a base, preferably DMAP. The resulting mixture is then stirred for 12 to 24 hours, preferably for about 24 hours, at room temperature, preferably at about 25°C. The compound of formula (Ib) is then isolated from the reaction mixture by isolation techniques standard in the art of peptide chemistry, for example, evaporation of solvents, extraction, flash chromatography and/or HPLC.

The compound of formula (Ib) is then hydrolyzed under mild acidic conditions, preferably with trifluoroacetic acid, to yield a compound of formula (Ic).

The resulting compound of formula (Ic) is then treated with *O*-benzylhydroxylamine under standard amide coupling conditions to form a compound of formula (Id). For example, a cold (0-5°C) solution of the compound of formula (Ic) and HOBT in an inert solvent, preferably DMF, is treated with an excess molar amount of EDCI. After stirring the resulting mixture for about 30 minutes to an hour at 0-5°C, preferably at 0°C, an equimolar amount of *O*-benzylhydroxyamine is added. The reaction mixture is allowed to warm to room temperature overnight. The compound of formula (Id) is then isolated from the reaction mixture by standard isolation techniques, for example, by extraction and flash chromatography.

The hydroxyl-protecting group (benzyl) of the compound of formula (Id) is then removed under catalytic hydrogenation conditions (Pd/C) to yield a compound of formula (Ie).

**E. Preparation of Compounds of Formula (G)**

Compounds of formula (G):

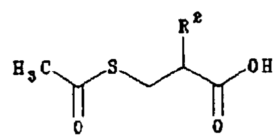

(G)

wherein $R^2$ is as defined above in the Summary of the Invention, are intermediates in the preparation of compounds of formula (I) and are prepared as shown in the following Reaction Scheme 5 where $R^2$ is as defined above in the Summary of the Invention.

**Reaction Scheme 5**

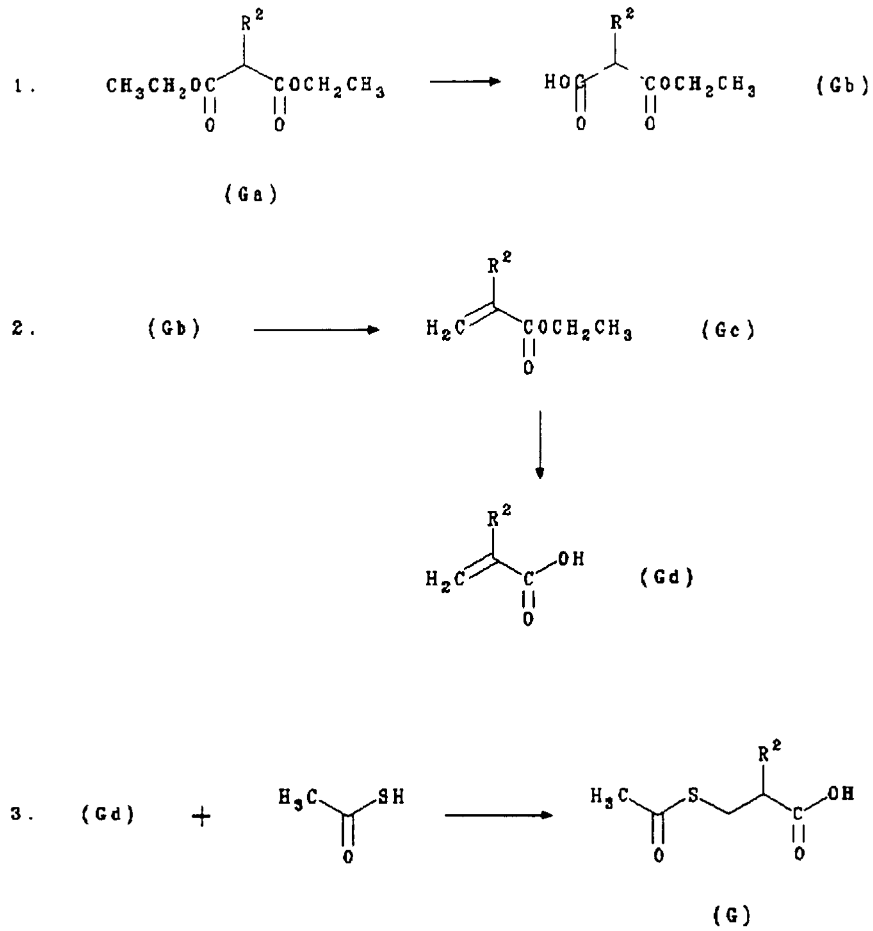
1. (Ga) (Gb)
2. (Gb) (Gc) (Gd)
3. (Gd) + (G)

Compounds of formula (Ga) and thioacetic acid are commercially available, for example, from TCI America Organic Chemicals and the Aldrich Chemical Company, respectively.

In general, a compound of formula (G) is prepared by first hydrolyzing a compound of formula (Ga) with an equimolar amount of a base, for example, potassium hydroxide, to yield a compound of formula (Gb). The compound of formula (Gb) is then deprotonated under basic conditions, for example, in the presence of triethylamine, at 0-5°C, preferably at 0°C, and then reacted with formaldehyde, followed by treatment with aqueous base, preferably potassium carbonate, to yield a compound of formula (Gc), which is isolated from the reaction mixture by standard isolation procedures.

The compound of formula (Gc) is then hydrolyzed under basic conditions, preferably in the presence of lithium hydroxide, to yield a compound of formula (Gd). The compound of formula (Gd) is then reacted with an excess molar amount of thioacetic acid at 90-100°C, preferably at 95°C, under an inert atmosphere. The compound of formula (G) is then isolated from the reaction mixture by standard isolation techniques, for example, by extraction and evaporation.

**F. Preparation of Compounds of Formulae (If) and (Ig)**

Compounds of formula (If) are compounds of formula (I) wherein $R^1$ is acetylthio; and $R^2$, $R^3$, $R^4$ and $R^5$ are as defined above in the Summary of the Invention.

Compounds of formula (Ig) are compounds of formula (I) wherein $R^1$ is mercapto; and $R^2$, $R^3$, $R^4$ and $R^5$ are as defined above in the Summary of the Invention.

Compounds of formulae (If) and (Ig) are prepared as shown in following Reaction Scheme 6 wherein $R^2$, $R^3$, $R^4$ and $R^5$ are as defined in the Summary of the Invention:

**Reaction Scheme 6**

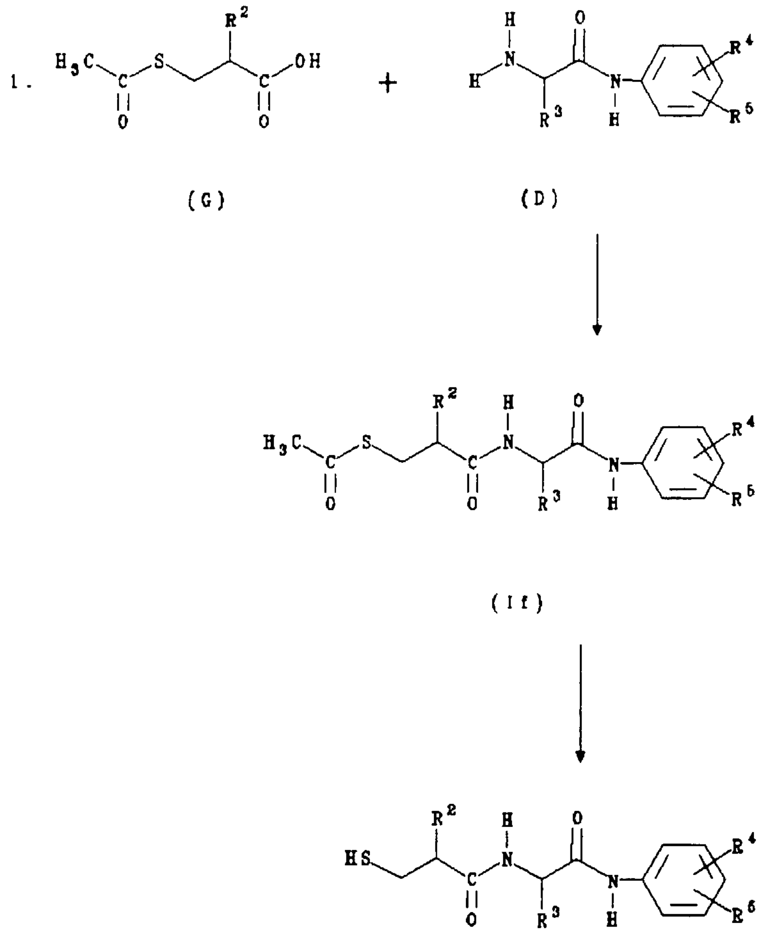

1.

(G) (D)

(If)

(Ig)

Compounds of formula (D) are prepared as shown above in Section B. Compounds of formula (G) are prepared as shown above in Section E.

In general, compounds of formulae (If) and (Ie) are prepared by first coupling a compound of formula (G) with a compound of formula (D) under standard amide coupling conditions to yield a compound of formula (If). For example, to a solution of the compound of formula (G) and HOBT in an aprotic solvent, preferably DMF, is added an excess molar amount of EDCI. The resulting mixture is stirred overnight at room temperature. The resulting compound of formula (If) is then isolated from the reaction mixture by standard isolation techniques, for example, by evaporation of solvent, extraction, and flash chromatography. The compound of formula (If) is then hydrolyzed under basic conditions, preferably in a protic solvent such as methanol in the presence of ammonium hydroxide, to form a compound of formula (Ig).

In addition, all compounds of formula (I) that exist in either the free acid or the free base form may be converted to their pharmaceutically acceptable salts by treatment with the appropriate inorganic or organic base or with the appropriate inorganic or organic acid, respectively. Salts of the compounds of formula (I) can also be converted to the free acid or free base form or to another salt.

In summary, compounds of formulae (Ia), (Ib), (Ic), (Id), (Ie), (If) and (Ig), which are all compounds of formula (I), are prepared by:

1. reacting a compound of formula (D) where $R^3$ and $R^4$ are as defined above in the Summary of the Invention; and $R^5$ is hydrogen; with a compound of formula (E) where $R^6$ is optionally substituted aryl, wherein the aryl group

is quinol-2-yl, naphth-1-yl, naphth-2-yl, pyridyl or phenyl; and $R^2$ is alkyl; to form a compound of formula (Ia) wherein $R^1$ is

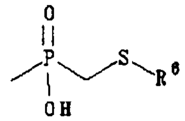

where $R^6$ is as defined above for the compound of formula (E); and $R^2$, $R^3$, $R^4$, and $R^5$ are as defined above for the compounds of formulae (D) or (E); or

2. reacting a compound of formula (F) wherein $R^2$ is as defined above in the Summary of the Invention; and $R^8$ is alkyl or benzyl; with a compound of formula (D) wherein $R^3$, $R^4$ and $R^5$ are as defined above in the Summary of the Invention; to form a compound of formula (Ib) wherein $R^1$ is alkoxycarbonyl or aralkoxycarbonyl; and $R^2$, $R^3$, $R^4$, $R^5$ and $R^8$ are as defined above for the compounds of formulae (F) or (D); or

3. treating a compound of formula (Ib) wherein $R^2$, $R^3$, $R^4$, and $R^5$ are as defined above in the Summary of the Invention; and $R^8$ is alkyl or benzyl; to form a compound of formula (Ic) wherein $R^1$ is carboxy; and $R^2$, $R^3$, $R^4$, and $R^5$ are as defined above in the Summary of the Invention; or

4. reacting a compound of formula (Ic) wherein $R^2$, $R^3$, $R^4$, and $R^5$ are as defined above in the Summary of the Invention; with *O*-benzylhydroxyamine to form a compound of formula (Id) wherein $R^1$ is benzyloxycarbamoyl; and $R^2$, $R^3$, $R^4$, and $R^5$ are as defined above in the Summary of the Invention; or

5. treating a compound of formula (Id) wherein $R^2$, $R^3$, $R^4$, and $R^5$ are as defined above in the Summary of the Invention; to form a compound of formula (Ie) wherein $R^1$ hydroxycarbamoyl; and $R^2$, $R^3$, $R^4$, and $R^5$ are as defined above in the Summary of the Invention; or

6. reacting a compound of formula (G) wherein $R^2$ is as defined above in the Summary of the Invention; with a compound of formula (D) wherein $R^3$, $R^4$ and $R^5$ are as defined above in the Summary of the Invention; to form a compound of formula (If) wherein $R^1$ is acetylthio; and $R^2$, $R^3$, $R^4$ and $R^5$ are as defined above in the Summary of the Invention; or

7. treating a compound of formula (If) wherein $R^2$, $R^3$, $R^4$ and $R^5$ are as defined above in the Summary of the Invention; to form a compound of formula (Ig) wherein $R^1$ is mercapto; and $R^2$, $R^3$, $R^4$ and $R^5$ are as defined above in the Summary of the Invention.

The following specific preparations and examples are provided as a guide to assist in the practice of the invention, and are not intended as a limitation on the scope of the invention.

**Example 1**

Compounds of formula (Ea)

A. Crystalline phosphinic acid (8.4 g, 0.13 mol) was stirred in neat triethylorthoformate (22 mL, 0.20 mL) for 90 minutes at room temperature. This was then transferred via cannula to a stirred solution of ethylisobutylacrylate (8 g, 0.036 mol) and tetramethylguanidine (4.5 mL, 0.036 mol) that had been cooled to 0°C for 10 minutes. The ice bath was removed and the reaction stirred for 4 hours. The mixture was diluted with 200 mL of diethyl ether and the solution washed with 1 N HCl (100 mL), water (4x100 mL), brine (100 mL), and dried over magnesium sulfate. This was rotary-evaporated to yield 8.15 g of 2-(ethoxy)phosphinoylmethyl-4-methylpentanoic acid ethyl ester as a slightly yellow colored oil, MS: 349 (M- $H_2O)^+$.

B. In a similar manner, the following compounds of formula (Ea) are prepared:

2-(ethoxy)phosphinoylmethyl-5-phenylpentanoic acid ethyl ester;
2-(ethoxy)phosphinoylmethyl-4-phenylbutanoic acid ethyl ester;
2-(ethoxy)phosphinoylmethyl-3-phenylpropanoic acid ethyl ester;
2-(ethoxy)phosphinoylmethyl-3-cyclohexylpropanoic acid ethyl ester; and
2-((ethoxy)phosphinoylmethyl)pentanoic acid ethyl ester.

**Example 2**

Compound of formula (Eb)

A. Crude 2-(ethoxy)phosphinoylmethyl-4-methylpentanoic acid ethyl ester (26 g) was dissolved in 600 mL THF/ $CH_2Cl_2$ (50/50) and cooled to 0°C. *N,N*-diisopropylethylamine (32 mL) and 90.8 mL of *bis*-(trimethylsilyl) acetamide were added to the solution and the resulting mixture was stirred for 20 minutes and then paraformaldehyde (5.5 g) was added. The solution was brought to room temperature and heated at 37°C for 18 hours. The solvent was removed by evaporation, and the resulting oil dissolved in 200 mL ethyl acetate. The solution was washed with 50 mL of 1N HCl (2x), 50 mL of brine (2x), dried over $MgSO_4$, filtered and evaporated to yield 19.3 g of 2-(ethoxy)(hydroxymethyl)phosphinoylmethyl-4-methylpentanoic acid ethyl ester as a faintly yellow oil, MS: 281.2 ($MH^+$).

B. In a similar manner, the following compounds of formula (Eb) are prepared:

2-(ethoxy)(hydroxymethyl)phosphinoylmethyl-5-phenylpentanoic acid ethyl ester;
2-(ethoxy)(hydroxymethyl)phosphinoylmethyl-4-phenylbutanoic acid ethyl ester;
2-(ethoxy)(hydroxymethyl)phosphinoylmethyl-3-phenylpropanoic acid ethyl ester;
2-(ethoxy)(hydroxymethyl)phosphinoylmethyl-3-cyclohexyl-propanoic acid ethyl ester; and
2-((ethoxy)(hydroxymethyl)phosphinoylmethyl)pentanoic acid ethyl ester.

**Example 3**

Compounds of formula (Ec)

A. 2-(Ethoxy) (hydroxymethyl)phosphinoylmethyl-4-methylpentanoic acid ethyl ester (5 g) was dissolved in 20 mL of $CH_2Cl_2$ and cooled to -20°C (in duplicate). Methanesulfonyl chloride (1.5 mL) and triethylamine (3.0 mL) were added dropwise to the solution. After 15 minutes the bath was removed and the reaction left at room temperature for 3½ hours. Each solution was then washed with 10 mL cold 2% HCl, 10 mL $NaHCO_3$ (sat), 10 mL brine, dried with $MgSO_4$, filtered and evaporated to yield 12.8 g (combined yield) of 2-(ethoxy)(methanesulfonyloxymethyl) phosphinoylmethyl-4-methylpentanoic acid ethyl ester.

B. In a similar manner, but replacing methanesulfonyl chloride with p-toluenesulfonyl chloride, 2-(ethoxy)-(*p*-toluenesulfonyloxymethyl)-phosphinoylmethyl-4-methylpentanoic acid ethyl ester is prepared.

C. In a similar manner, the following compounds of formula (Ec) are prepared:

2-(ethoxy)(methanesulfonyloxymethyl)phosphinoylmethyl-5-phenylpentanoic acid ethyl ester;
2-(ethoxy)(methanesulfonyloxymethyl)phosphinoylmethyl-4-phenylbutanoic acid ethyl ester;
2-(ethoxy)(methanesulfonyloxymethyl)phosphinoylmethyl-3-phenylpropanoic acid ethyl ester;
2-(ethoxy)(methanesulfonyloxymethyl)phosphinoylmethyl-3-cyclohexylpropanoic acid ethyl ester;
2-((ethoxy)(methanesulfonyloxymethyl)phosphinoylmethyl)-pentanoic acid ethyl ester;
2-(ethoxy)(*p*-toluenesulfonyloxymethyl)phosphinoylmethyl-5-phenylpentanoic acid ethyl ester;
2-(ethoxy)(*p*-toluenesulfonyloxymethyl)phosphinoylmethyl-4-phenylbutanoic acid ethyl ester;
2-(ethoxy)(*p*-toluenesulfonyloxymethyl)phosphinoylmethyl-3-phenylpropanoic acid ethyl ester;
2-(ethoxy)(*p*-toluenesulfonyloxymethyl)phosphinoylmethyl-3-cyclohexylpropanoic acid ethyl ester; and
2-((ethoxy)(*p*-toluenesulfonyloxymethyl)phosphinoylmethyl)-pentanoic acid ethyl ester.

**Example 4**

Compounds of formula (Ee)

Sodium hydride (1.52 g, (60%)) and 2-quinolinethiol (6 g) were stirred together at 0°C in 50 mL DMF. After the initial $H_2$ evolution had subsided, the mixture was stirred at room temperature for 2.5 hours. The mixture was then cooled to 0°C and 2-(ethoxy)(methanesulfonyloxymethyl)-phosphinoylmethyl-4-methylpentanoic acid ethyl ester (12.8 g) in 10 mL DMF was added via cannula. The resulting mixture was stirred for 18 hours, slowly warming to room temperature. The DMF was removed by evaporation, the residue dissolved in 50 mL ethyl acetate and washed with 50 mL $H_2O$ (2x), brine (50 mL), dried over $MgSO_4$ and evaporated to a yellow semisolid. Purification by flash chromatography using 10% ethyl acetate/hexane to 80% ethyl acetate/hexane for the elution yielded 10 g of 2-(ethoxy)-(quinolin-2-ylthiomethyl)phosphinoylmethyl-4-methylpentanoic acid ethyl ester (Rf 0.35 80% ethyl acetate/hexane), MS: 424.1 ($MH^+$).

B. In a similar manner, but replacing 2-quinolinethiol with 1-naphthalenethiol, 2-naphthalenethiol or thiophenol, the following compounds of formula (Ee) are prepared:

2-(ethoxy)(naphth-1-ylthiomethyl)phosphinoylmethyl-4-methylpentanoic acid ethyl ester;
2-(ethoxy)(naphth-2-ylthiomethyl)phosphinoylmethyl-4-methylpentanoic acid ethyl ester; and
2- (ethoxy) (phenylthiomethyl)phosphinoylmethyl-4-methylpentanoic acid ethyl ester.

C. In a similar manner, the following compounds of formula (Ee) are prepared:

2-(ethoxy)(quinolin-2-ylthiomethyl)phosphinoylmethyl-5-phenylpentanoic acid ethyl ester;
2-(ethoxy)(quinolin-2-ylthiomethyl)phosphinoylmethyl-4-phenylbutanoic acid ethyl ester;
2-(ethoxy)(quinolin-2-ylthiomethyl)phosphinoylmethyl-3-phenylpropanoic acid ethyl ester;
2-(ethoxy)(quinolin-2-ylthiomethyl)phosphinoylmethyl-3-cyclohexylpropanoic acid ethyl ester;
2-((ethoxy) (quinolin-2-ylthiomethyl)phosphinoylmethyl)-pentanoic acid ethyl ester;
2-(ethoxy)(naphth-1-ylthiomethyl)phosphinoylmethyl-5-phenylpentanoic acid ethyl ester;
2-(ethoxy)(naphth-1-ylthiomethyl)phosphinoylmethyl-4-phenylbutanoic acid ethyl ester;
2-(ethoxy)(naphth-1-ylthiomethyl)phosphinoylmethyl-3-phenylpropanoic acid ethyl ester;
2-(ethoxy)(naphth-1-ylthiomethyl)phosphinoylmethyl-3-cyclohexylpropanoic acid ethyl ester;
2-((ethoxy) (naphth-1-ylthiomethyl)phosphinoylmethyl)-pentanoic acid ethyl ester;
2-(ethoxy)(naphth-2-ylthiomethyl)phosphinoylmethyl-5-phenylpentanoic acid ethyl ester;
2-(ethoxy)(naphth-2-ylthiomethyl)phosphinoylmethyl-4-phenylbutanoic acid ethyl ester;
2-(ethoxy)(naphth-2-ylthiomethyl)phosphinoylmethyl-3-phenylpropanoic acid ethyl ester;
2-(ethoxy)(naphth-2-ylthiomethyl)phosphinoylmethyl-3-cyclohexylpropanoic acid ethyl ester;
2-((ethoxy) (naphth-2-ylthiomethyl)phosphinoylmethyl)-pentanoic acid ethyl ester;
2-(ethoxy)(phenylthiomethyl)phosphinoylmethyl-5-phenylpentanoic acid ethyl ester;
2-(ethoxy)(phenylthiomethyl)phosphinoylmethyl-4-phenylbutanoic acid ethyl ester;
2-(ethoxy)(phenylthiomethyl)phosphinoylmethyl-3-phenylpropanoic acid ethyl ester;
2-(ethoxy)(phenylthiomethyl)phosphinoylmethyl-3-cyclohexylpropanoic acid ethyl ester; and
2- ((ethoxy) (phenylthiomethyl)phosphinoylmethyl)-pentanoic acid ethyl ester.

**Example 5**

Compounds of formula (E)

A. 2-(Ethoxy) (quinolin-2-ylthiomethyl)phosphinoyl-methyl-4-methylpentanoic acid ethyl ester (4.5 g) was dissolved in 100 mL THF and 12.5 mL of 2N NaOH was added together with enough methanol to make the solution homogeneous. After 18 hours the THF was removed by evaporation, the residue diluted with 50 mL $H_2O$ and washed with 50 mL ethyl acetate. The aqueous phase was then acidified to pH 4, and the product extracted with 50 mL ethyl acetate (2x). The ethyl acetate was washed with 20 mL brine, dried with $MgSO_4$ and evaporated to yield 3.8 g of 2-(hydroxy)-(quinolin-2-ylthiomethyl)phosphinoylmethyl-4-methylpentanoic acid as a yellow oil, MS: 368 ($MH^+$).

B. In a similar manner, the following compounds of formula (E) are prepared:

2-(hydroxy)(naphth-1-ylthiomethyl)phosphinoylmethyl-4-methylpentanoic acid;
2 - (hydroxy) (naphth-2-ylthiomethyl)phosphinoylmethyl-4-methylpentanoic acid; and
2 - (hydroxy) (phenylthiomethyl)phosphinoylmethyl-4-methylpentanoic acid.

C. In a similar manner, the following compounds of formula (E) are prepared:

2 - (hydroxy) (quinolin-2-ylthiomethyl)phosphinoylmethyl-5-phenylpentanoic acid;
2 - (hydroxy) (quinolin-2-ylthiomethyl)phosphinoylmethyl-4-phenylbutanoic acid;
2 - (hydroxy) (quinolin-2-ylthiomethyl)phosphinoylmethyl-3-phenylpropanoic acid;
2-(hydroxy)(quinolin-2-ylthiomethyl)phosphinoylmethyl-3-cyclohexylpropanoic acid;
2-((hydroxy) (quinolin-2-ylthiomethyl)phosphinoylmethyl)-pentanoic acid;
2 - (hydroxy) (naphth-1-ylthiomethyl)phosphinoylmethyl-5-phenylpentanoic acid;
2-(hydroxy)(naphth-1-ylthiomethyl)phosphinoylmethyl-4-phenylbutanoic acid;
2-(hydroxy)(naphth-1-ylthiomethyl)phosphinoylmethyl-3-phenylpropanoic acid;
2-(hydroxy)(naphth-1-ylthiomethyl)phosphinoylmethyl-3-cyclohexylpropanoic acid;

2-((hydroxy)(naphth-1-ylthiomethyl)phosphinoylmethyl)-pentanoic acid;
2-(hydroxy)(naphth-2-ylthiomethyl)phosphinoylmethyl-5-phenylpentanoic acid;
2-(hydroxy)(naphth-2-ylthiomethyl)phosphinoylmethyl-4-phenylbutanoic acid;
2-(hydroxy)(naphth-2-ylthiomethyl)phosphinoylmethyl-3-phenylpropanoic acid;
2 - (hydroxy) (naphth-2-ylthiomethyl)phosphinoylmethyl-3-cyclohexylpropanoic acid;
2-((hydroxy)(naphth-2-ylthiomethyl)phosphinoylmethyl)-pentanoic acid;
2- (hydroxy) (phenylthiomethyl)phosphinoylmethyl-5-phenylpentanoic acid;
2-(hydroxy)(phenylthiomethyl)phosphinoylmethyl-4-phenylbutanoic acid;
2-(hydroxy)(phenylthiomethyl)phosphinoylmethyl-3-phenylpropanoic acid;
2-(hydroxy)(phenylthiomethyl)phosphinoylmethyl-3-cyclohexylpropanoic acid; and
2- ((hydroxy) (phenylthiomethyl)phosphinoylmethyl)pentanoic acid.

**Example 6**

Resolution of a compound of formula (E)

2 - (Hydroxy) (quinolin-2-ylthiomethyl)phosphinoyl-methyl-4-methylpentanoic acid (5.3 g) was dissolved in 50 mL of warm ethanol (abs) and 4.2 g of (-)-cinchonidine was added. After 30 minutes at room temperature the salt began to precipitate out. The flask was covered in foil and allowed to stand for 2 days. The salt was then removed by suction filtration, and the filtrate evaporated to a yellow foam. The salt and the filtrate were each dissolved in 100 mL ethyl acetate and washed successively with 1% HCl to remove the cinchonidine while keeping the pH above 4. Both solutions were each dried over $MgSO_4$ and evaporated to yield 2.4 g of a single stereoisomer, $[\alpha]_D^{24}$=+10.68° (9.73 mg in methanol (2 mL)) and 2.5 g of the other single stereoisomer, $[\alpha]_D^{24}$=-8.70° (9.88 mg in methanol (2 mL)).

**Example 7**

Compounds of formula (B)

A. To a cold (0°C) suspension of 4-acetamidobenzenesulphonyl chloride (4.0 g, 17 mmol) in $CH_2Cl_2$ (40 mL) was added pyridine (1.7 mL, 20 mmol) and DMAP (209 mg, 1.7 mmol). A clear solution resulted. Anhydrous methylamine was bubbled into the solution for 1 hour at 0°C, and then the solution was allowed to stir at 25°C for 2 hours. The solution was extracted with 1M NaOH (3x15 mL) and the combined extracts were adjusted to pH 6 at 0°C with 3M HCl. The product, which precipitated as fluffy white crystals, was filtered and washed with cold water to afford 3.2 g (82%) of 4-acetamido-*N*-methylbenzenesulphonamide; $^1$H NMR (300 MHz, MeOH) δ 2.35 (s,3H), 2.70 (s,3H), 7.96 (s,4H).

B. A mixture of 4-acetamido-*N*-methylbenzenesulphonamide (3.2 g, 14 mmol) and 100 mL of 1M HC1 was refluxed under argon for 3 hours. After cooling to 25°C, $CH_2Cl_2$ (10 mL) was added and the aqueous phase was neutralized with 1M NaOH at 0°C. The aqueous phase was separated and extracted with $CH_2Cl_2$ (2x25 mL). The combined organic phases were washed with brine (10 mL), dried ($Na_2SO_4$) and concentrated to afford 1.5 g (58%) of a compound of formula (B) where $R^4$ is *N*-methylsulfonamide as a colorless solid; $^1$H NMR (300 MHz, MeOH) δ 2.46 (s,3H), 6.67-6.72 ($AA^1$ part of $AA^1XX^1$ 2H), 7.48-7.52 ($XX^1$ part of $AA^1XX^1$, 2H).

**Example 8**

Compounds of formula (C)

A. To a cold (0°C) solution of *N-tert*-butoxycarbonyl-L-leucine (1.4 g, 6.3 mmol) and HOBT (1.5 g, 9.8 mmol) in DMF (30 mL) was added EDCI (2.5 g, 14 mmol) in portions. After stirring for 1 hour at 0°C, the resulting solution was treated with methyl 4-aminobenzoate (1.09 mL, 6.8 mmol) and DMAP (0.32 g, 2.6 mmol). After stirring for 24 hours at 25°C, the DMF was removed *in vacuo.* The residue was dissolved in $CH_2Cl_2$ and washed with saturated $NaHCO_3$ solution, 1M HCl (twice), and brine. Drying over $Na_2SO_4$ and concentration *in vacuo* afforded the crude product which was purified by flash chromatography on $SiO_2$ (20% ethyl acetate/hexanes eluent). There was obtained 1.0 g (85%) of *N-t*-butoxycarbonyl-L-leucine-*N'*-(4-methoxycarbonylphenyl)carboxamide as a foamy solid, MS (FAB) 363 $(M-H)^-$.

B. In a similar manner, the following compounds of formula (C) were prepared:

*N-t*-butoxycarbonyl-L-tryptophan-*N'*-phenylmethylcarboxamide;
*N-t*-butoxycarbonyl-L-tryptophan-*N'*-phenylcarboxamide;

*N-t*-butoxycarbonyl-L-tryptophan-*N'*-(4-methoxycarbonylphenyl)carboxamide;
*N-t*-butoxycarbonyl-L-tryptophan-*N'*-(4-ethoxycarbonylphenyl)carboxamide;
*N-t*-butoxycarbonyl-L-leucine-*N'*-(4-(*N"*-methylaminosulfonyl)phenyl)-carboxamide;
*N-t*-butoxycarbonyl-L-alanine-*N'*-(4-methoxycarbonylphenyl)carboxamide;
*N-t*-butoxycarbonyl-L-methionine-*N'*-(4-methoxycarbonylphenyl)carboxamide;
*N-t*-butoxycarbonyl-L-leucine-*N'*-(3-ethoxycarbonylphenyl)carboxamide;
*N-t*-butoxycarbonyl-L-leucine-*N'*-(2-methoxycarbonylphenyl)carboxamide;
*N-t*-butoxycarbonyl-L-leucine-*N'*-(4-(1-methylethyloxy)carbonyl)phenyl)-carboxamide;
*N-t*-butoxycarbonyl-L-leucine-*N'*-(aminosulfonyl)phenyl)carboxamide;
*N-t*-butoxycarbonyl-L-leucine-*N'*-(4-methoxycarbonylmethylphenyl)-carboxamide;
*N-t*-butoxycarbonyl-L-pyridin-3-ylalanine-*N'*-(4-methoxycarbonylphenyl)-carboxamide;
*N-t*-butoxycarbonyl-L-cyclohexylglycine-*N'*-(4-methoxycarbonylphenyl)-carboxamide;
*N-t*-butoxycarbonyl-L-isoleucine-*N'*-(4-methoxycarbonylphenyl)carboxamide;
*N-t*-butoxycarbonyl-L-O-benzylthreonine-*N'*-(4-methoxycarbonylphenyl)-carboxamide;
*N-t*-butoxycarbonyl-L-*t*-leucine-*N'*-(4-methoxycarbonylphenyl)carboxamide;
*N*- *t*-butoxycarbonyl-L-leucine-*N'*-(4-cyanophenyl)carboxamide;
*N-t*-butoxycarbonyl-L-leucine-*N'*-(4-(*N"*-(2-dimethylaminoethylcarbamoyl)-carboxamide; and
*N-t*-butoxycarbonyl-L-leucine-*N'*-(4-(*N"*-(3-dimethylaminopropyl)carbamoyl)-phenyl)carboxamide.

C. In a similar manner, the following compounds of formula (C) are prepared:

*N-t*-butoxycarbonyl-L-tryptophan-*N'*-(4-nitrophenyl)carboxamide;
*N-t*-butoxycarbonyl-L-tryptophan-*N'*-(4-aminophenyl)carboxamide;
*N-t*-butoxycarbonyl-L-leucine-*N'*-(4-methylsulfonylphenyl)carboxamide;
*N-t*-butoxycarbonyl-L-leucine-*N'*-(4-ethylsulfonylphenyl)carboxaanide; and
*N-t*-butoxycarbonyl-L-leucine-*N'*-(4-tetrazolylphenyl)carboxamide.

**Example 9**

Compounds of formula (D)

A. To a cold (0°C) solution of *N-t*-butoxycarbonyl-L-leucine-*N'*-phenylcarboxamide (3.4 g, 11 mmol) in dry $CH_2Cl_2$ (10 mL) was added TFA (2 mL). The solution was allowed to stir at 25°C for 6 hours and was then concentrated *in vacuo*. The residue was partitioned between $CH_2Cl_2$ and $H_2O$ and the aqueous layer was basified at 0°C with saturated $K_2CO_3$ solution. The organic phase was separated and the aqueous layer was extracted three times with $CH_2Cl_2$. The combined organic layers were washed with brine and dried over $Na_2SO_4$. Concentration afforded L-leucine-*N'*-phenylcarboxamide.

B. In a similar manner, the following compounds are prepared:

L-leucine-*N'*-(4-methoxycarbonylphenyl)carboxamide;
L-tryptophan-*N'*-phenylmethylcarboxamide;
L-tryptophan-*N'*-phenylcarboxamide;
L-tryptophan-*N'*-(4-methoxycarbonylphenyl)carboxamide;
L-tryptophan-*N'*-(4-ethoxycarbonylphenyl)carboxamide;
L-leucine-*N'*-(4-(*N"*-methylaminosulfonyl)phenyl)carboxamide;
L-alanine-*N'*-(4-methoxycarbonylphenyl)carboxamide;
L-methionine-*N'*-(4-methoxycarbonylphenyl)carboxamide;
L-leucine-*N'*-(3-ethoxycarbonylphenyl)carboxamide;
L-leucine-*N'*-(2-methoxycarbonylphenyl)carboxamide;
L-leucine-*N'*-(4-(1-methylethyloxy)carbony)phenyl)carboxamide;
L-leucine-*N'*-(aminosulfonyl)phenyl)carboxamide;
L-leucine-*N'*-(4-methoxycarbonylmethylphenyl)carboxamide;
L-pyridi*n*-3-ylalanine-*N'*-(4-methoxycarbonylphenyl)carboxamide;
L-spirocyclopentylglycine-*N'*-(4-methoxycarbonylphenyl)carboxamide;
L-cyclohexylglycine-*N'*-(4-methoxycarbonylphenyl)carboxamide;
L-isoleucine-*N'*-(4-methoxycarbonylphenyl)carboxamide;
L-*O*-benzylthreonine-*N'*-(4-methoxycarbonylphenyl)carboxamide;
L-*t*-leucine-*N'*-(4-methoxycarbonylphenyl)carboxamide;

L-leucine-*N'*-(4-cyanophenyl)carboxamide;
L-leucine-*N'*-(4-(*N"*-(2-dimethylaminoethyl)carbamoyl)phenyl)carboxamide; and
L-leucine-*N'*-(4-(*N"*-(3-dimethylaminopropyl)carbamoyl)phenyl)carboxamide.

C. In a similar manner, the following compounds of formula (D) are prepared:

L-tryptophan-*N'*-(4-nitrophenyl)carboxamide;
L-tryptophan-*N'*-(4-aminophenyl)carboxamide;
L-leucine-*N'*-(4-methylsulfonylphenyl)carboxamide;
L-leucine-*N'*-(4-ethylsulfonylphenyl)carboxamide;and
L-leucine-*N'*-(4-tetrazolylphenyl)carboxamide.

**Example 10**

Compounds of formula (Ia)

A. To a cold (0°C) solution of 2-(hydroxy)(quinolin-2-ylthiomethyl)phosphinoylmethyl-4-methylpentanoic acid (0.20 g, 0.54 mmol) in THF (6 mL) was added 1,1'-carbonyldiimidazole (0.12 g, 0.7 mmol). The mixture was stirred for 75 minutes at 0°C and was then treated with L-tryptophan-*N'*-(4-ethoxycarbonylphenyl)carboxamide (0.22 g, 0.62 mmol) and stirred at 25°C for 15 hours. The THF was evaporated and the residue was dissolved in ethyl acetate (60 mL). The solution was washed with $H_2O$ (10 mL), brine (10 mL), and dried over $MgSO_4$. Concentration was followed by reverse phase HPLC using a gradient of acetonitrile and 50 mM $NH_4OAc$ buffer afforded 30 mg of *N*-(2-(hydroxy)(quinolin-2-ylthiomethyl)phosphinoyl-methyl-4-methylpentanoyl)-L-tryptophan-*N'*-(4-ethoxycarbonylphenyl)carboxamide as an off white solid, MS(FAB) 701 $(M-H)^+$ (mixture of diastereomers).

B. In a similar manner, the following compounds of formula (Ia) were prepared:

*N*-(2-(hydroxy)(quinolin-2-ylthiomethyl)phosphinoylmethyl-4-methylpentanoyl)-L-tryptophan-*N'*-(4-methoxycarbonylphenyl)carboxamide,
MS(FAB) 687 $(M+H)^+$;
*N*-(2-(hydroxy)(quinolin-2-ylthiomethyl)phosphinoylmethyl-4-methylpentanoyl)-L-alanine-*N'*-(4-methoxycarbonylphenyl)carboxamide,
MS(FAB) 572 $(M+H)^+$;
*N*-(2-(hydroxy)(quinolin-2-ylthiomethyl)phosphinoylmethyl-4-methylpentanoyl)-L-methionine-*N'*-(4-methoxycarbonylphenyl)carboxamide,
MS(FAB) 632 $(M+H)^+$; **Compound 1**
*N*-(2-(hydroxy)(quinolin-2-ylthiomethyl)phosphinoylmethyl-4-methylpentanoyl)-L-leucine-*N'*-(4-methoxycarbonylphenyl)carboxamide,
MS(FAB) 614 $(M+H)^+$;
*N*-(2-(hydroxy)(quinolin-2-ylthiomethyl)phosphinoylmethyl-4-methylpentanoyl)-L-leucine-*N'*-(3-ethoxycarbonylphenyl)carboxamide,
$^1H$ NMR (300 MHz, MeOH) δ 0.73-1.01 (m, 12H), 1.28-2.00 (m, 14H), 2.4-3.61 (m, 2H), 4.27-4.45 (m, 3H), 7.23-7.44 (m, 3H), 7.65-7.98 (m, 6H), 8.29 (s, 0.5H), 8.50 (s, 0.5H);
*N*-(2-(hydroxy)(quinolin-2-ylthiomethyl)phosphinoylmethyl4-methylpentanoyl)-L-leucine-*N'*-(2-methoxycarbonylphenyl)carboxamide,
$^1H$ NMR (300 MHz, MeOH) δ 0.78-0.99 (m, 13H), 1.3-2.4 (m, 7H), 2.90-3.05 (m, 1H), 3.5-3.75 (m, 2H), 3.89, 3.90, 3.94 (3s, 3H total), 4.35-3.50 (m, 1H), 7.05-8.10 (m, 11H), 8.32, 8.55, 8.60 (3d, J=8.7, 1H);
*N*-(2-(hydroxy)(quinolin-2-ylthiomethyl)phosphinoylmethyl-4-methylpentanoyl)-L-leucine-*N'*-(4-(1,1-dimethylethoxycarbonyl-phenyl)carboxamide, MS(FAB) 642 $(MH)^+$;
*N*-(2-(hydroxy)(quinolin-2-ylthiomethyl)phosphinoylmethyl4-methylpentanoyl)-L-leucine-*N'*-(4-aminosulfonylphenyl)carboxamide,
$^1H$ NMR (300 MHz, MeOH) δ 0.76 (d, J=6.5, 3H), 0.81 (d, J=6.5, 3H), 0.85-1.1 (m, 7H), 1.2-2.1 (m, 7H), 2.92-2.95 (m, 1H), 3.45-3.70 (m, 2H), 4.35-4.45 (m, 1H), 7.28 (d, J=8.7, 1H), 7.45 (t, J=8.7, 1H), 7.68 (t, J=8.7, 1H), 7.7-7.8 (m, 3H), 7.87 (d, J=8.7, 1H), 7.95-8.1 (m, 3H);
*N*-(2-(hydroxy)(quinolin-2-ylthiomethyl)phosphinoylmethyl-4-methylpentanoyl)-L-leucine-*N'*-(4-methoxycarbonylmethylphenyl)carboxamide,
MS(FAB) 628 $(MH)^+$.

C. In a similar manner, the following compounds of formula (Ia) are prepared:

*N*-(2-(hydroxy)(quinolin-2-ylthiomethyl)phosphinoylmethyl-5-phenylpentanoyl)-L-leucine-*N'*-(4-methoxycarbonylphenyl)carboxamide;
*N*-(2-(hydroxy)(quinolin-2-ylthiomethyl)phosphinoylmethyl-4-phenylbutanoyl)-L-leucine-*N'*-(4-methoxycarbonylphenyl)carboxamide;
*N*-(2-(hydroxy)(quinolin-2-ylthiomethyl)phosphinoylmethyl-3-phenylpropanoyl)-L-leucine-*N'*-(4-methoxycarbonylphenyl)carboxamide;
*N*-(2-(hydroxy)(quinolin-2-ylthiomethyl)phosphinoylmethyl-3-cyclohexylpropanoyl)-L-leucine-*N'*-(4-methoxycarbonylphenyl)carboxamide;
*N*-(2-((hydroxy)(quinolin-2-ylthiomethyl)phosphinoylmethyl)pentanoyl)-L-leucine-*N'*-(4-methoxycarbonylphenyl)carboxamide;
*N*-(2-(hydroxy)(naphth-1-ylthiomethyl)phosphinoylmethyl-5-phenylpentanoyl)-L-leucine-*N'*-(4-methoxycarbonylphenyl)carboxamide;
*N*-(2-(hydroxy)(naphth-1-ylthiomethyl)phosphinoylmethyl-4-phenylbutanoyl)-L-leucine-*N'*-(4-methoxycarbonylphenyl)carboxamide;
*N*-(2-(hydroxy)(naphth-1-ylthiomethyl)phosphinoylmethyl-3-phenylpropanoyl)-L-leucine-*N'*-(4-methoxycarbonylphenyl)carboxamide;
*N*-(2-(hydroxy)(naphth-1-ylthiomethyl)phosphinoylmethyl-3-cyclohexylpropanoyl)-L-leucine-*N'*-(4-methoxycarbonylphenyl)carboxamide;
*N*-(2-((hydroxy)(naphth-1-ylthiomethyl)phosphinoylmethyl)pentanoyl)-L-leucine-*N'*-(4-methoxycarbonylphenyl)carboxamide;
*N*-(2-(hydroxy)(naphth-2-ylthiomethyl)phosphinoylmethyl-5-phenylpentanoyl)-L-leucine-*N'*-(4-methoxycarbonylphenyl)carboxamide;
*N*-(2-(hydroxy)(naphth-2-ylthiomethyl)phosphinoylmethyl-4-phenylbutanoyl)-L-leucine-*N'*-(4-methoxycarbonylphenyl)carboxamide;
*N*-(2-(hydroxy)(naphth-2-ylthiomethyl)phosphinoylmethyl-3-phenylpropanoyl)-L-leucine-*N'*-(4-methoxycarbonylphenyl)carboxamide;
*N*-(2-(hydroxy)(naphth-2-ylthiomethyl)phosphinoylmethyl-3-cyclohexyl-propanoyl)-L-leucine-*N'*-(4-methoxycarbonylphenyl)carboxamide;
*N*-(2-((hydroxy)(naphth-2-ylthiomethyl)phosphinoylmethyl)pentanoyl)-L-leucine-*N'*-(4-methoxycarbonylphenyl)carboxamide;
*N*-(2-(hydroxy)(phenylthiomethyl)phosphinoylmethyl-5-phenylpentanoyl)-L-leucine-*N'*-(4-methoxycarbonylphenyl)carboxamide;
*N*-(2-(hydroxy)(phenylthiomethyl)phosphinoylmethyl-4-phenylbutanoyl)-L-leucine-*N'*-(4-methoxycarbonylphenyl)carboxamide;
*N*-(2-(hydroxy)(phenylthiomethyl)phosphinoylmethyl-3-phenylpropanoyl)-L-leucine-*N'*-(4-methoxycarbonylphenyl)carboxamide;
*N*-(2-(hydroxy)(phenylthiomethyl)phosphinoylmethyl-3-cyclohexylpropanoyl)-L-leucine-*N'*-(4-methoxycarbonylphenyl)carboxamide;and
*N*-(2-((hydroxy)(phenylthiomethyl)phosphinoylmethyl)pentanoyl)-L-leucine-*N'*-(4-methoxycarbonylphenyl) carboxamide.

D. A solution of *N*-(2-(hydroxy)(quinolin-2-ylthiomethyl)-phosphinoylmethyl-4-methylpentanoyl)-L-tryptophan-*N'*-(4-methoxycarbonylphenyl)carboxamide in THF (2 mL) and 1M NaOH (1 mL) was stirred for 24 hours at 25°C. The organic solvents were evaporated, and the residue dissolved in ethyl acetate/$H_2O$. The aqueous phase was acidified with 1M HCl and the separated aqueous phase was extracted twice with ethyl acetate. The combined organic layers were washed with brine, dried ($MgSO_4$) and concentrated to 27 mg of *N*-(2-(hydroxy)(quinoli*n*-2-ylthiomethyl)-phosphinoylmethyl-4-methylpentanoyl)-L-tryptophan-*N'*-(4-carboxyphenyl)-carboxamide as a yellow powder.
E. In a similar manner, but starting with *N*- (2- (hydroxy) (quinolin-2-ylthiomethyl)phosphinoylmethyl-4-methylpentanoyl)-L-leucine-*N'*-(4-methoxycarbonylphenyl)carboxamide (30 mg, 0.048 mmol), there was obtained 10 mg of *N*-(2-(hydroxy)(quinolin-2-ylthiomethyl)phosphinoylmethyl-4-methylpentanoyl)-L-leucine-*N'*-(4-carboxyphenyl) carboxamide as a semisolid after trituration with ethyl acetate;
$^1$H NMR[1] (300 MHZ, MeOH) 0.81-1.02 (m, 12H), 1.1-2.3 (m, 10H), 2.82-3.00 (m, 1H), 3.49, 3.56 (2s,2H), 3.5-3.8 (m,2H), 4.45-4.55 (m,1H), 7.09 (d, J=8.2, 1H), 7.19 (d, J=8.2,1H), 7.45 (t, J=8.2, 1H) 7.45-7.6 (m, 3H), 7.65-7.80 (m, 1H), 7.82-7.98 (m, 2H), 8.10-8.20 (m, 1H).

**Example 11**

Compounds of formula (Fa)

A. To 4-methylpentanoic acid (25 g, 0.215 mmol) in a 25°C water bath, thionyl chloride (20.4 mL, 1.3 g) was slowly added. The mixture was heated at 50°C under argon for 3 hours (until the evolution of gas had stopped). The crude reaction mixture was distilled at atmospheric pressure to give 4-methylpentanoyl chloride (25.3 g, 87.3%), b.p. 143°C.
B. In a similar manner, but replacing 4-methylpentanoic acid with 5-phenylpentanoic acid (5 g), 5-phenylpentanoyl chloride was prepared (4.4 g), as a colorless liquid, b.p. 91°-93°C.

**Example 12**

Compounds of formula (Fb)

A. To a suspension of 60% NaH (836 mg, 1.5 eq.) in toluene (200 mL) at room temperature under argon was added L- (+)-2,10-camphor sultam (3.0 g, 3.9 mmol) portion-wise. The mixture was stirred vigorously at room temperature for one hour. Then 4-methylpentanoyl chloride was carefully added dropwise to the solution at 0°C. After stirring the reaction at room temperature for 3 hours, the reaction was quenched with 10 mL of water, and 70 mL of ether was added. The reaction mixture was first washed with 0.5N HCl (2x50 mL), then 5% $K_2CO_3$ (3x50 mL) and finally with brine (1x50 mL). The organic layer was dried over $MgSO_4$, filtered and evaporated to dryness. Purification by column chromatography (1:6 ethyl acetate/petroleum ether as eluant) gave *N*-4-methylpentanoyl-L-(+)-2,10-camphor sultam (3.39 g, 78%).
B. In a similar manner, but replacing 4-methylpentanoyl chloride with the appropriate chloride, the following compounds of formula (Fb) were prepared:

*N*-3-phenylpropanoyl-L-(+)-2,10-camphor sultam, MS: 347 ($M^+$);
*N*-5-phenylpentanoyl-L-(+)-2,10-camphor sultam, MS: 375 $M^+$;
*N*-pentanoyl-L-(+)-2,10-camphor sultam, MS: 300 $(M+H)^+$.

**Example 13**

Compounds of formula (Fc)

A. To a solution of *N*-4-methylpentanoyl-L-(+)-2,10-camphor sultam (3.39 g, 10.8 mmol) in 75 mL of dry THF at -78°C under argon was added $NaN(TMS)_2$ (1.0 M in THF, 11.34 mL, 1.05 eq.) dropwise over five minutes. After stirring at -78°C for 1 hour, hexamethylphosphoramide (5 mL) was added to the mixture, followed by *t*-butylbromoacetate (5.2 ml, 3 eq), then 400 mg of tetra *n*-butyl ammonium iodide was added in one portion. The resulting solution was kept at -78°C under argon overnight. The next morning, the reaction was quenched with water (100 mL) and then extracted with ether (3x100 mL). The combined ether layers were washed with brine, dried over $Na_2SO_4$, filtered and concentrated. Purification by column chromatography (5:95 ethyl acetate/petroleum ether to 10:90 ethyl acetate/petroleum ether as eluant) gave *N*-(4-methyl-2-*t*-butoxycarbonylmethyl)pentanoyl-L- (+) -2,10-camphor sultam (4 g, 86.5%).
B. In a similar manner, but replacing *N*-4-methylpentanoyl-L-(+)-2,10-camphor sultam with the appropriate compound of formula (Fb), the following compounds of formula (Fc) were prepared:

*N*- (3-phenyl-2- *t*-butoxycarbonylmethyl)propanoyl-L-(+)-2,10-camphor sultam, MS: 461 ($M^+$);
*N*-(5-phenyl-2-*t*-butoxycarbonylmethyl)pentanoyl-L- (+)-2,10-camphor sultam, MS: 490.1 $(M+H)^+$;
*N*-(2-*t*-butoxycarbonylmethyl)pentanoyl-L-(+)-2,10-camphor sultam, MS: 414 $(M+H)^+$.

**Example 14**

Compounds of formula (F)

A. To a stirred solution of *N*-(4-methyl-2-*t*-butoxycarbonylmethyl) pentanoyl-L-(+)-2,10-camphor sultam (5.45 g,

12.7 mmol) in 50% aqueous THF (150 mL) at 0°C under argon was added LiOH·$H_2O$ crystals (2.14 g, 4 eq.) followed by 30% $H_2O_2$ (11.5 mL). The ice-bath was removed and the resulting emulsion was stirred for 3 hours before it turned clear. Most of the THF was removed under reduced pressure at 35°C. $CH_2Cl_2$ (150 mL) was added and then 4N HCl with stirring to pH=2. After adding NaCl, the aqueous layer was extracted with $CH_2Cl_2$ (3x150 mL). The $CH_2Cl_2$ was removed under reduced pressure at 35°C. The residue was taken up in ethyl acetate (150 mL). This solution was then extracted with 5% $K_2CO_3$ (3x50 mL) and the combined extracts were washed with ether (50 mL). $CH_2Cl_2$ was added to the aqueous layer and then NaCl with stirring. The aqueous layer was extracted with $CH_2Cl_2$ (3x70 mL) and the combined extracts were dried over $Na_2SO_4$, filtered and concentrated to give (2*R*)-4-methyl-2-*t*-butoxycarbonylmethylpentanoic acid as a colorless oil (2.95 g, quantitative yield).

B. In a similar manner, but replacing *N*-(4-methyl-2-*t*-butoxycarbonylmethyl)pentanoyl-L-(+)-2,10-camphor sultam with the appropriate compound of formula (Fc), the following compounds of formula (F) were prepared:

(2*R*)-3-phenyl-2-*t*-butoxycarbonylmethyl-propanoic acid, MS: 265 $(M+H)^+$;
(2*R*)-5-phenyl-2-*t*-butoxycarbonylmethyl-pentanoic acid, MS: 293.1 $(M+H)^+$;
(2*R*)-2-*t*-butoxycarbonylmethyl-pentanoic acid, (colorless oil, 1.09 g).

C. (2*R*)-3-Phenyl-2-*t*-butoxycarbonylmethyl-propanoic acid (55 mg) was taken up in glacial acetic acid (20 mL) and $PtO_2$ (25 mg) was added in acetic acid. The beaker was placed in a Parr bomb, it was evacuated and charged with 100 psi of $H_2$. After stirring for 3 days, the mixture was suction filtered through a 1 cm bed of celite. The filtrate was then concentrated to a yellow oil, (2*R*)-3-cyclohexyl-2-*t*-butoxycarbonylmethylpropanoic acid (56 mg), MS: 269.5 $(M-H)^-$.

**Example 15**

Compounds of formula (Ib)

A. To a solution of 4-methyl-2-*t*-butoxycarbonylmethylpentanoic acid (0.28 g, 1.2 mmol) in DMF (5 mL) containing HOBT (0.22 g, 1.8 mmol) was added EDCI (0.31 g, 1.8 mmol). The mixture was stirred at 0°C for 1 hour and was then treated with L-cyclohexylglycine-*N'*-(4-methoxycarbonylphenyl)carboxamide (1.2 mmol) and DMAP (27 mg, 0.24 mmol). Stirring was continued for 24 hours at 25°C and then the DMF was evaporated. The residue was dissolved in $CH_2Cl_2$ (20 mL) and the solution was washed with 1M HCl (10 mL), saturated $NaHCO_3$ (10 mL), brine (10 mL) and dried over $Na_2SO_4$. Concentration *in vacuo* afforded an oil which was purified by flash chromatography on $SiO_2$ using 20% ethyl acetate/hexenes as eluent.
There was obtained 0.22 g (22%) of *N*-(4-methyl-2-*t*-butoxycarbonylmethylpentanoyl)-L-cyclohexylglycine-*N'*-(4-methoxycarbonylphenyl) carboxamide as a solid, MS(FAB) 503 $(MH)^+$.
B. In a similar manner, the following compounds were prepared:

*N*-(4-methyl-2-*t*-butoxycarbonylmethylpentanoyl)-L-pyridin 3-ylalanine-*N'*-(4-methoxycarbonylphenyl)carboxamide;
*N*-(4-methyl-2-*t*-butoxycarbonylmethylpentanoyl)-L-*O*-benzyl-threonine-*N'*-(4-methoxycarbonylphenyl)carboxamide;
*N*-(4-methyl-2-*t*-butoxycarbonylmethylpentanoyl)-L-isoleucine-N'-(4-methoxycarbonylphenyl)carboxamide;
*N*-(4-methyl-2-*t*-butoxycarbonylmethylpentanoyl)-L-leucine-*N'*-(4-methoxycarbonylphenyl)carboxamide;
*N*-(4-methyl-2-*t*-butoxycarbonylmethylpentanoyl)-L-*t*-leucine-*N'*-(4-methoxycarbonylphenyl)carboxamide;
*N*-(4-methyl-2-*t*-butoxycarbonylmethylpentanoyl)-L-leucine-*N'*-(4-cyanophenyl)carboxamide;
*N*-(4-methyl-2-*t*-butoxycarbonylmethylpentanoyl)-L-leucine-*N'*-(4-(*N"*-(3-dimethylaminopropyl)carbamoyl) phenyl)-carboxamide;
*N*-(4-methyl-2-*t*-butoxycarbonylmethylpentanoyl)-L-leucine-*N'*-(4-(*N"*-(2-dimethylaminoethyl)carbamoyl)phenyl)-carboxamide;
*N*-(4-methyl-2-*t*-butoxycarbonylmethylpentanoyl)-L-leucine-*N'*-(4-aminosulfonylphenyl)carboxamide;
*N*-(4-methyl-2-*t*-butoxycarbonylmethylpentanoyl)-L-leucine-*N'*-(4-methylaminosulfonylphenyl)carboxamide;
*N*-(2-*t*-butoxycarbonylmethylpentanoyl)-L-leucine-*N'*-(4-methoxycarbonylphenyl)carboxamide;
*N*-(3-phenyl-2-*t*-butoxycarbonylmethylpropanoyl)-L-leucine-*N'*-(4-methoxycarbonylphenyl)carboxamide;
*N*-(3-cyclohexyl-2-*t*-butoxycarbonylmethylpropanoyl)-L-leucine-*N'*-(4-methoxycarbonylphenyl)carboxamide;
*N*-(4-phenyl-2-*t*-butoxycarbonylmethylbutanoyl)-L-leucine-*N'*-(4-methoxycarbonylphenyl)carboxamide;and
*N*-(5-phenyl-2-*t*-butoxycarbonylmethylpentanoyl)-L-leucine-*N'*-(4-methoxycarbonylphenyl)carboxamide.

**Example 16**

Compounds of formula (Ic)

A. To a cold (0°C) solution of *N*-(4-methyl-2-*t*-butoxycarbonylmethylpentanoyl)-L-cyclohexylglycine-*N'*-(4-methoxycarbonylphenyl)-carboxamide (70 mg, 0.14 mmol) in $CH_2Cl_2$ (2 mL) was added TFA (0.5 mL). After stirring for 5 hours at 25°C, the solution was concentrated *in vacuo* and the product was purified by reverse phase HPLC using a gradient of acetonitrile and 50 mM $NH_4OAc$ buffer to provide 44 mg (71%) of *N*-(4-methyl-2-carboxymethylpentanoyl)-L-cyclohexylglycine-*N'*-(4-methoxycarbonylphenyl)-carboxamide as a white solid, MS(FAB) 445 $(M-H)^-$.

B. In a similar manner, the following compounds were prepared:

*N*-(4-methyl-2-carboxymethylpentanoyl)-L-isoleucine-*N'*-(4-methoxycarbonyl-phenyl)carboxamide, MS(FAB) 419 $(M-H)^-$;
*N*-(4-methyl-2-carboxymethylpentanoyl)-L-leucine-*N'*-(4-methoxycarbonylphenyl)carboxamide, MS(FAB) 419 $(M-H)^-$;
*N*-(4-methyl-2-carboxymethylpentanoyl)-L-*t*-leucine-*N'*-(4-methoxycarbonylphenyl)carboxamide;
*N*-(4-methyl-2-carboxymethylpentanoyl)-L-leucine-*N'*-(4-cyanophenyl)carboxamide,
$^1H$ NMR (300 MHz, MeOH) δ 0.84-0.99 (m, 12H), 1.15-1.82 (m, 6H), 2.36-2.41 (m, 1H), 2.52-2.65 (m, 1H), 2.8-2.95 (m, 1H), 4.49-4.54 (m, 1H), 7.4-7.9 (m, 4H);
*N*-(4-methyl-2-carboxymethylpentanoyl)-L-leucine-*N'*-(4-aminosulfonylphenyl)carboxamide, $^1H$ NMR (300 MHZ, MeOH) 0.85-1.00 (m, 12H), 1.1-1.3 (m, 2H), 1.52-1.85 (m, 4H), 2.31-2.95 (m, 3H), 4.49-4.55 (m, 1H), 7.75-7.91 (m, 4H);
*N*-(4-methyl-2-carboxymethylpentanoyl)-L-leucine-*N'*-(4-methylaminosulfonylphenyl)carboxamide, MS(FAB) 459 $(M-H)^-$;
*N*-(2-carboxymethylpentanoyl)-L-leucine-*N'*-(4-methoxycarbonylphenyl)carboxamide, MS(FAB) 405 $(M-H)^-$;
*N*-(3-phenyl-2-carboxymethylpropanoyl)-L-leucine-*N'*-(4-methoxycarbonylphenyl)carboxamide, MS(FAB) 455 $(M+H)^+$;
*N*-(3-cyclohexyl-2-carboxymethylpropanoyl)-L-leucine-*N'*-(4-methoxycarbonylphenyl)carboxamide, MS(FAB) 459 $(M-H)^-$;
*N*-(4-phenyl-2-carboxymethylbutanoyl)-L-leucine-*N'*-(4-methoxycarbonylphenyl)carboxamide, MS(FAB) 467 $(N\text{-}H)^-$;
*N*-(4-phenyl-2-carboxymethylbutanoyl)-L-cyclohexylglycine-*N'*-(4-methoxycarbonylphenyl)carboxamide;
*N*-(4-phenyl-2-carboxymethylbutanoyl)-L-*t*-leucine-*N'*-(4-methoxycarbonylphenyl)carboxamide;
*N*-(5-phenyl-2-carboxymethylpentanoyl)-L-leucine-*N'*-(4-methoxycarbonylphenyl)carboxamide, MS(FAB) 481 $(M-H)^-$; and
*N*-(4-methyl-2-carboxymethylpentanoyl)-L-*O*-benzylthreonine-*N'*-(4-methoxycarbonylphenyl) carboxamide, MS(FAB) 497 $(M-H)^-$.

C. In a similar manner, but triturating the crude product with ether and then decanting the ether to yield the following compounds as TFA salts:

*N*-(4-methyl-2-carboxymethylpentanoyl)-L-pyridin-3-ylalanine-*N'*-(4-methoxycarbonylphenyl)carboxamide, MS(FAB) 456 $(M+H)^+$;
*N*-(4-methyl-2-carboxymethylpentanoyl)-L-leucine-*N'*-(4-(*N''*-(3-dimethylaminopropyl)carbamoyl)phenyl)carboxamide, MS(FAB) 491 $(M+H)^+$; and
*N*-(4-methyl-2-carboxymethylpentanoyl)-L-leucine-*N'*-(4-(*N''*-(2-dimethylaminoethyl)carbamoyl)phenyl)carboxamide, MS(FAB) 491 $(M+H)^+$.

D. A mixture of *N*-(4-methyl-2-*t*-butoxycarbonylmethyl-pentanoyl)-L-*O*-benzylthreonine-*N'*-(4-methoxycarbonylphenyl)-carboxamide (60 mg) and Pd/C in ethyl acetate/THF (1:1, 25 mL) was hydrogenated overnight at 1 atm pressure. Filtration through Celite, concentration of the filtrate, and trituration of the residue with ether/hexenes produced *N*-(4-methyl-2-*t*-butoxycarbonylmethyl-pentanoyl)-L-threonine-*N'*-(4-methoxycarbonylphenyl)-carboxamide, MS(FAB) 407 $(M-H)^-$.

**Example 17**

Compounds of formula (Id)

A. A solution of *N*-(4-methyl-2-carboxymethylpentanoyl)-L-leucine-*N'*-(4-methoxycarbonylphenyl)carboxamide (0.28 g, 0.66 mmol) and HOBT (0.12 g) in dry DMF (20 mL) was cooled to 0°C and treated with EDCI (0.32 g). After stirring 0.5 hours at 0°C, *O*-benzylhydroxylamine (0.30 mL) was added and the reaction was allowed to warm to 25°C overnight. The DMF was removed *in vacuo* and the residue was taken up in $CH_2Cl_2$ and washed with 5% HCl/5% $NaHCO_3$ and brine and the solution was dried over $Na_2SO_4$. After concentration, the product was purified by flash chromatography ($SiO_2$, $R_f$=0.6, 10% MeOH/$CH_2Cl_2$). The product containing fractions were further purified by trituration with $CH_2Cl_2$ to give *N*-(4-methyl-2- *(N"*-benzyloxycarbamoyl)methylpentanoyl)-L-leucine-*N'*-(4-methoxycarbonylphenyl)-carboxamide as a solid, mp 198-199°C.

B. In a similar manner, the following compounds were prepared:

*N*-(2-(*N"*-benzyloxycarbamoyl)methylpentanoyl)-L-leucine-*N'*-(4-methoxycarbonylphenyl)carboxamide;
*N*-(4-phenyl-2-(*N"*-benzyloxycarbamoyl)methylbutanoyl)-L-leucine-*N'*-(4-methoxycarbonylphenyl)carboxamide; and
*N*-(4-methyl-2-(*N"*-benzyloxycarbamoyl)methylpentanoyl)-L-tryptophan-*N'*-(4-methoxycarbonylphenyl)carboxamide.

C. *N*-(4-Methyl-2-(*N"*-benzyloxycarbamoyl)methylpentanoyl)-L-leucine-*N'*-(4-methoxycarbonylphenyl)-carboxamide (210 mg) was hydrolyzed with 1M NaOH (1.4 mL) at 50-60°C for 2 hour in THF (20 mL) and MeOH (5 mL). The organic solvents were evaporated and the residue was taken up in 10 mL $H_2O$ and washed with ether (2x10 mL). The aqueous phase was acidified to pH 2 with 10% HCl and extracted with ethyl acetate (3x10 mL). The combined extracts were washed with brine, dried over $Na_2SO_4$, and concentrated to afford *N*-(4-methyl-2-(*N"*-benzyloxycarbamoyl)methylpentanoyl)-L-leucine-*N'*-(4-carboxyphenyl)carboxamide (110 mg).

**Example 18**

Compounds of formula (Ie)

A. To a solution of *N*-(4-phenyl-2-(*N"*-benzyloxycarbamoyl)-methylbutanoyl)-L-leucine-*N'*-(4-methoxycarbonylphenyl)carboxamide (25 mg) in 20 mL MeOH and 10 mL THF was added 10% Pd/C (20 mg). The suspension was hydrogenated for 1 hour and then suction filtered through Celite. Concentration afforded the product which was purified on silica (2.5% MeOH/$CH_2Cl_2$) to give 8 mg of *N*-(4-phenyl-2-(*N"*-hydroxycarbamoyl)-methylbutanoyl)-L-leucine-*N'*-(4-methoxycarbonylphenyl)carboxamide, MS(FAB) 482 $(M-H)^-$.

B. In a similar manner, the following compounds were prepared:

*N*-(4-methyl-2-(*N"*-hydroxycarbamoyl)methylpentanoyl)-L-leucine-*N'*-(4-carboxyphenyl)carboxamide;
*N*-(4-methyl-2-(*N"*-hydroxycarbamoyl)methylpentanoyl)-L-leucine-*N'*-(4-methoxycarbonylphenyl)carboxamide, MS(FAB) 436 $(M+H)^+$; Compound 2
*N*-(2-(*N'*-hydroxycarbamoyl)methylpentanoyl)-L-leucine-*N'*-(4-methoxycarbonylphenyl)carboxamide, MS (FAB) 420 $(M-H)^-$;
*N*-(4-phenyl-2-(*N"*-hydroxycarbamoyl)methylbutanoyl)-L-*t*-leucine-*N'*-(4-methoxycarbonylphenyl)carboxamide;
*N*-(4-phenyl-2-(*N"*-hydroxycarbamoyl)methylbutanoyl)-L-cyclohexylglycine-*N'*-(4-methoxycarbonylphenyl) carboxamide;
*N*-(4-phenyl-2-(*N"*-hydroxycarbamoyl)methylbutanoyl)-L-leucine-*N'*-(4-methoxycarbonylphenyl)carboxamide;
*N*-(4-methyl-2-(*N"*-hydroxycarbamoyl)methylpentanoyl)-L-*t*-leucine-*N'*-(4-methoxycarbonylphenyl)carboxamide;
*N*-(4-methyl-2-(*N"*-hydroxycarbamoyl)methylpentanoyl)-L-tryptophan-*N'*-(4-methoxycarbonylphenyl)carboxamide, MS(FAB) 507 $(M-H)^-$;
**Compound 3 (2*R*); Compound 4 (2S); and**
*N*-(4-phenyl-2-(*N"*-hydroxycarbamoyl)methylbutanoyl)-L-leucine-*N'*-(4-methoxycarbonylphenyl)carboxamide.

C. In a similar manner, the following compounds are prepared:

*N*-(3-phenyl-2-(*N*″-hydroxycarbamoyl)methylpropanoyl)-L-leucine-*N*′-(4-methoxycarbonylphenyl)carboxamide;
*N*-(5-phenyl-2-(*N*″-hydroxycarbaanoyl)methylpentanoyl)-L-leucine-*N*′-(4-methoxycarbonylphenyl)carboxamide; and
*N*-(3-cyclohexyl-2-(*N*″-hydroxycarbamoyl)methylpropanoyl)-L-leucine-*N*′-(4-methoxycarbonylphenyl)carboxamide.

**Example 19**

Compounds of formula (Gb)

A. To a cold (0°C) solution of diethyl isobutylmalonate (21.6 g, 0.1 mol) in 150 mL of ethanol was added a solution of KOH (5.89 g, 0.1 mol) slowly over 30 minutes. The clear solution was stirred at 25°C for 60 hours. The ethanol was removed under reduced pressure and the solid residue was dissolved in 50 mL of $H_2O$. The aqueous solution was acidified to pH 2 with 4M HCl and extracted with ether (2x50 mL). The combined extracts were dried over $MgSO_4$ and evaporated to provide 19.0 g (100%) of ethyl isobutylmalonate as a colorless oil.
B. In a similar manner, the following compounds of formula (Gb) are prepared:

ethyl ter*t*-butylmalonate;
ethyl propylmalonate;
ethyl benzylmalonate; and
ethyl cyclohexymethylmalonate.

**Example 20**

Compounds of formulae (Gc) and (Gd)

A. To neat ethyl isobutylmalonate (25 g, 0.13 mol) at 0°C was slowly added ice cold diethylamine (15.1 mL, 0.15 mol). After stirring for 15 minutes, formalin (11.1 mL of 37% aqueous formaldehyde) was added dropwise and the mixture was allowed to stir at 25°C for 3 days. The reaction was treated with a solution of 20 g of $K_2CO_3$ in 40 mL of $H_2O$ and extracted with ether (2 x 100 mL). The combined ether layers were washed with brine, dried over $MgSO_4$, and evaporated at 20°C on a rotary evaporator. The crude product ethyl 4-methyl-2-methylenepentanoate (containing some ether) was dissolved in 250 mL of absolute ethanol and treated with acetonitrile (250 mL), 1M LiOH (9.7 g in 250 mL of $H_2O$, 0.23 mol). After stirring overnight, the organic solvents were evaporated and the aqueous residue was extracted with ethyl acetate (2x150 mL). The combined extracts were washed with brine, dried ($MgSO_4$), and evaporated to afford 10.5 g of 4-methyl-2-methylenepentanoic acid as a colorless oil.
B. In a similar manner, the following compounds of formula (Gd) are prepared:

4-phenyl-2-methylenebutanoic acid;
3-cyclohexyl-2-methylenepropanoic acid;
5-phenyl-2-methylenepentanoic acid;
2-methylenepentanoic acid; and
3,3-dimethyl-2-methylenebutanoic acid.

**Example 21**

Compounds of formula (G)

A. A mixture of 4-methyl-2-methylenepentanoic acid (5.0 g) and thioacetic acid (25 mL) was heated at 95°C under argon for 3 days. The excess thioacetic acid was evaporated and the residual oil was dissolved in ethyl acetate (40 mL) and extracted with saturated $NaHCO_3$ (3x40 mL). The combined $NaHCO_3$ extracts were combined and acidified at 0°C to pH 2 with 1M HCl. The aqueous layer was extracted with $CH_2Cl_2$ (3x40 mL), the combined organic phases were dried ($MgSO_4$), and finally evaporated to give 3.0 g of 4-methyl-2-acetylthiomethylpentanoic acid; $^1H$ NMR (80 MHz, $CDCL_3$) $\delta$ 0.95 (d, J=8.0, 6H), 1.20-1.90 (m, 4H), 2.35 (s, 3H), 2.50-3.20 (m, 3H), 6.7 (br s, 1H).

**Example 22**

Compounds of formula (If)

A. To a solution of 4-methyl-2-acetylthiomethylpentanoic acid (204 mg, 1.0 mmol) in dry DMF (15 mL) containing HOBT (92 mg, 0.6 mmol) and L-leucine-*N'*-(4-methoxycarbonylphenyl)carboxamide (0.6 mmol) was added EDCI (345 mg, 1.8 mmol). The solution was stirred overnight at 25°C and then the DMF was removed *in vacuo.* The residue was dissolved in ethyl acetate (35 mL) and washed with 1M HCl, 1M NaOH, and brine. Drying over $MgSO_4$ and evaporation afforded a semisolid which was flash chromatographed on silica gel (ethyl acetate 1:petroleum ether 2) to give *N*-(4-methyl-2-acetylthiomethylpentanoyl)-L-leucine-*N'*-(4-methoxycarbonylphenyl)carboxamide (190 mg) as a white solid.

B. In a similar manner, the following compounds of formula (If) are prepared:

*N*-(5-phenyl-2-acetylthiomethylpentanoyl)-L-leucine-*N'*-(4-methoxycarbonylphenyl)carboxamide;
*N*-(4-phenyl-2-acetylthiomethylbutanoyl)-L-leucine-*N'*-(4-methoxycarbonylphenyl)carboxamide;
*N*-(3-phenyl-2-acetylthiomethylpropanoyl)-L-leucine-*N'*-(4-methoxycarbonylphenyl)carboxamide;
*N*-(3-cyclohexyl-2-acetylthiomethylpropanoyl)-L-leucine-*N'*-(4-methoxycarbonylphenyl)carboxamide;
*N*-(2-acetylthiomethylpentanoyl)-L-leucine-*N'*-(4-methoxycarbonylphenyl)carboxamide;
*N*-(5-phenyl-2-acetylthiomethylpentanoyl)-L-leucine-*N'*-(4-aminocarbonylphenyl)carboxamide;
*N*-(4-phenyl-2-acetylthiomethylbutanoyl)-L-leucine-*N'*-(4-carboxyphenyl)carboxamide;
*N*-(3-phenyl-2-acetylthiomethylpropanoyl)-L-leucine-*N'*-(4-methylsulfonylphenyl)carboxamide;
*N*-(3-cyclohexyl-2-acetylthiomethylpropanoyl)-L-leucine-*N'*-(4-carbamoylphenyl)carboxamide;
*N*-(2-acetylthiomethylpentanoyl)-L-leucine-*N'*-(4-cyanophenyl)carboxamide;
*N*-(5-phenyl-2-acetylthiomethylpentanoyl)-L-tryptophan-*N'*-(4-methoxycarbonylphenyl)carboxamide;
*N*-(4-phenyl-2-acetylthiomethylbutanoyl)-L-tryptophan-*N'*-(4-methoxycarbonylphenyl)carboxamide;
*N*-(3-phenyl-2-acetylthiomethylpropanoyl)-L-tryptophan-*N'*-(4-methoxycarbonylphenyl)carboxamide;
*N*-(3-cyclohexyl-2-acetylthiomethylpropanoyl)-L-tryptophan-*N'*-(4-methoxycarbonylphenyl)carboxamide; and
*N*-(2-acetylthiomethylpentanoyl)-L-tryptophan-*N'*-(4-methoxycarbonylphenyl)carboxamide.

**Example 23**

Compounds of formula (Ig)

A. To a solution of *N*-(4-methyl-2-acetylthiomethylpentanoyl)-L-leucine-*N'*-(4-methoxycarbonylphenyl)carboxamide (85 mg, 0.19 mmol) in MeOH (8 mL) at O°C was added concentrated $HN_4OH$ (0.4 mL). After stirring at O°C for 5 hours, the methanol was evaporated and ether (30 mL) was added. The ether solution was washed with 0.5 M HCl, brine, and was dried over $MgSO_4$. Concentration afforded *N*-(4-methyl-2-mercaptomethylpentanoyl)-L-leucine-*N'*-(4-methoxycarbonylphenyl)carboxamide in quantitative yield as a white foam, MS(FAB) 407 $(M-H)^-$.

B. In a similar manner, the following compounds of formula (Ig) are prepared:

*N*-(5-phenyl-2-mercaptomethylpentanoyl)-L-leucine-*N'*-(4-methoxycarbonylphenyl)carboxamide;
*N*-(4-phenyl-2-mercaptomethylbutanoyl)-L-leucine-*N'*-(4-methoxycarbonylphenyl)carboxamide;
*N*-(3-phenyl-2-mercaptomethylpropanoyl)-L-leucine-*N'*-(4-methoxycarbonylphenyl)carboxamide;
*N*-(3-cyclohexyl-2-mercaptomethylpropanoyl)-L-leucine-*N'*-(4-methoxycarbonylphenyl)carboxamide;
*N*-(2-mercaptomethylpentanoyl)-L-leucine-*N'*-(4-methoxycarbonylphenyl)carboxamide;
*N*-(5-phenyl-2-mercaptomethylpentanoyl)-L-leucine-*N'*-(4-aminocarbonylphenyl)carboxamide;
*N*-(4-phenyl-2-mercaptomethylbutanoyl)-L-leucine-*N'*-(4-carboxyphenyl)carboxamide;
*N*-(3-phenyl-2-mercaptomethylpropanoyl)-L-leucine-*N'*-(4-methylsulfonylphenyl)carboxamide;
*N*-(3-cyclohexyl-2-mercaptomethylpropanoyl)-L-leucine-*N'*-(4-carbamoylphenyl)carboxamide;
*N*-(2-mercaptomethylpentanoyl)-L-leucine-*N'*-(4-cyanophenyl)carboxamide;
*N*-(5-phenyl-2-mercaptomethylpentanoyl)-L-tryptophan-*N'*-(4-methoxycarbonylphenyl)carboxamide;
*N*-(4-phenyl-2-mercaptomethylbutanoyl)-L-tryptophan-*N'*-(4-methoxycarbonylphenyl)carboxamide;
*N*-(3-phenyl-2-mercaptomethylpropanoyl)-L-tryptophan-*N'*-(4-methoxycarbonylphenyl)carboxamide;
*N*-(3-cyclohexyl-2-mercaptomethylpropanoyl)-L-tryptophan-*N'*-(4-methoxycarbonylphenyl)carboxamide; and
*N*-(2-mercaptomethylpentanoyl)-L-tryptophan-*N'*-(4-methoxycarbonylphenyl)carboxamide.

**Example 24**

This example illustrates the preparation of representative pharmaceutical compositions for oral administration containing a compound of formula (I), or a pharmaceutically acceptable salt thereof, *e.g.*, *N*-(4-methyl-2-(*N'*-hydroxycarbamoyl)methylpentanoyl)-L-leucine-*N'*-(4-methoxycarbonylphenyl)carboxamide:

| A. | Ingredients | % wt./wt. |
|---|---|---|
| | Compound of formula (I) | 20.0% |
| | Lactose | 79.5% |
| | Magnesium stearate | 0.5% |

The above ingredients are mixed and dispensed into hard-shell gelatin capsules containing 100 mg each, one capsule would approximate a total daily dosage.

| B. | Ingredients | % wt./wt. |
|---|---|---|
| | Compound of formula (I) | 20.0% |
| | Magnesium stearate | 0.9% |
| | Starch | 8.6% |
| | Lactose | 69.6% |
| | PVP (polyvinylpyrrolidine) | 0.9% |

The above ingredients with the exception of the magnesium stearate are combined and granulated using water as a granulating liquid. The formulation is then dried, mixed with the magnesium stearate and formed into tablets with an appropriate tableting machine.

| C. | Ingredients | |
|---|---|---|
| | Compound of formula (I) | 0.1 g |
| | Propylene glycol | 20.0 g |
| | Polyethylene glycol 400 | 20.0 g |
| | Polysorbate 80 | 1.0 g |
| | Water | q.s. 100 mL |

The compound of formula (I) is dissolved in propylene glycol, polyethylene glycol 400 and polysorbate 80. A sufficient quantity of water is then added with stirring to provide 100 mL of the solution which is filtered and bottled.

| D. | Ingredients | % wt./wt. |
|---|---|---|
| | Compound of formula (I) | 20.0% |
| | Peanut Oil | 78.0% |
| | Span 60 | 2.0% |

The above ingredients are melted, mixed and filled into soft elastic capsules.

**Example 25**

This example illustrates the preparation of a representative pharmaceutical formulation for parenteral administration containing a compound of formula (I), or a pharmaceutically acceptable salt thereof, e.g., *N*-(4-methyl-2-(*N"*-hydroxycarbamoyl)methylpentanoyl)-L-tryptophan-*N'*-(4-carboxyphenyl) carboxamide:

| Ingredients | |
|---|---|
| Compound of formula (I) | 0.02 g |
| Propylene glycol | 20.0 g |
| Polyethylene glycol 400 | 20.0 g |

(continued)

| Ingredients | |
|---|---|
| Polysorbate 80 | 1.0 g |
| 0.9% Saline solution | q.s. 100 mL |

The compound of formula (I) is dissolved in propylene glycol, polyethylene glycol 400 and polysorbate 80. A sufficient quantity of 0.9% saline solution is then added with stirring to provide 100 mL of the I.V. solution which is filtered through a 0.2 μ membrane filter and packaged under sterile conditions.

**Example 26**

This example illustrates the preparation of a representative pharmaceutical composition in suppository form containing a compound of formula (I), or a pharmaceutically acceptable salt thereof, e.g., *N*-(4-methyl-2-(*N"*-hydroxycarbamoyl)methylpentanoyl)-L-leucine-*N'*-(4-carboxyphenyl)carboxamide:

| Ingredients | % wt./wt. |
|---|---|
| Compound of formula (I) | 1.0% |
| Polyethylene glycol 1000 | 74.5% |
| Polyethylene glycol 4000 | 24.5% |

The ingredients are melted together and mixed on a steam bath, and poured into molds containing 2.5 g total weight.

**Example 27**

This example illustrates the preparation of a representative pharmaceutical formulation for insufflation containing a compound of formula (I), or a pharmaceutically acceptable salt thereof, e.g., *N*-(4-methyl-2-(*N"*-hydroxycarbamoyl) methylpentanoyl)-L-cyclohexylglycine-*N'*-(4-methoxycarbonyl-phenyl)carboxamide:

| Ingredients | % wt./wt. |
|---|---|
| Micronized compound of formula (I) | 1.0% |
| Micronized lactose | 99.0% |

The ingredients are milled, mixed, and packaged in an insufflator equipped with a dosing pump.

**Example 28**

This example illustrates the preparation of a representative pharmaceutical formulation in nebulized form containing a compound of formula (I), or a pharmaceutically acceptable salt thereof, *e.g.*, *N*-(4-methyl-2-(*N"*-hydroxycarbamoyl)methylpentanoyl)-L-*t*-leucine-*N'*-(4-methoxycarbonylphenyl)carboxamide:

| Ingredients | % wt./wt. |
|---|---|
| Compound of formula (I) | 0.005% |
| Water | 89.995% |
| Ethanol | 10.000% |

The compound of formula (I) is dissolved in ethanol and blended with water. The formulation is then packaged in a nebulizer equipped with a dosing pump.

**Example 29**

This example illustrates the preparation of a representative pharmaceutical formulation in aerosol form containing a compound of formula (I), or a pharmaceutically acceptable salt thereof, *e.g.*, *N*-(4-methyl-2-mercaptomethylpentanoyl)-L-leucine-*N'*-(4-methoxycarbonylphenyl)carboxamide:

| Ingredients | % wt./wt. |
|---|---|
| Compound of formula (I) | 0.10% |
| Propellant 11/12 | 98.90% |
| Oleic acid | 1.00% |

The compound of formula (I) is dispersed in oleic acid and the propellants. The resulting mixture is then poured into an aerosol container fitted with a metering valve.

**Example 30**

*In vitro* assay

Matrilysin was purified from cloned mammalian cell culture by Blue-Sepharose and zinc-chelating sepharose column followed by fast protein liquid chromatography over a MONO S column. The enzyme was activated by incubation with 1 mmol APMA for 1 hr at 35-37°C.

Compounds of formula (I) were dissolved in DMSO and added to a cuvette containing 0.4 µg matrilysin in 1 ml TC buffer (20mM Tris, 5mM $CaCl_2$, pH 7.5) (2% DMSO final concentration). The concentrations of the compounds of formula (I) were chosen such that there was at least one data point for every 20% change in activity. Enzyme and compounds were permitted to preincubate 3 min at 37°C. To initiate the reaction, *N*-(7-dimethylamino-4-methyl) coumarinyl ("DACM") (Sigma) and thiopeptide (Ac-Pro-Leu-Gly-S-"Leu"-Leu-Gly-OEt, Bachem Bioscience Inc.) were added to 20 µM each. The fluorescence increase was recorded with excitation and emission wavelengths of 395 and 485 nm, respectively. Each data point is the average of duplicate experiments. At least six data points, expressed as change in fluorescence per minute versus compound concentration were analyzed using the $IC_{50}$ fit in the program, Enzfitter.

Compounds of formula (I) exhibited the ability to inhibit matrilysin when tested in this assay.

| | **$IC_{50}$ (nM)** | | | |
|---|---|---|---|---|
| | **HFC** | **PUMP** | **HNG** | **STROM.** |
| Compound 2 | 8.4 | $k^i$=140fM | | 1.4 |

**Example 31**

*In vitro* assay

This assay determines if the compounds of formula (I) inhibit the release of $^{35}S$-labelled glycosaminoglycans (GAG's) from cartilage explants.

Small cartilage explants (3 mm diameter) were prepared from freshly sacrificed bovine knee joints and labeled with $^{35}SO_4$. $^{35}S$-labelled glycosaminoglycans (GAG's) are released into the culture medium in response to the addition of rhIL-1-alpha, which induces the expression of chondrocyte matrix metalloproteases (MMP's), including stromelysin and collagenase. The percent inhibition of labeled GAG's was corrected for spontaneous release in the absence of rh1L-1-alpha. Results for each group represent the mean ± the S.E.M. for five explants.

Compounds of formula (I), when tested in this assay, displayed the ability to inhibit the release of $^{35}S$-labelled GAG's from cartilage explants.

| | **$EC_{50}$ (molar)** |
|---|---|
| Compound 2 | $3.5\times10^{-6}$ |

**Example 32**

*In vitro* assay

An *in vitro* fetal rat long bone model was used to study the anti-bone resorptive effect of the compounds of formula (I). Bovine PTH was used to induce bone resorption *in vitro.* The bone resorptive effects were expressed by the amounts of $^{45}Ca$ released from the $^{45}Ca$ pre-labelled fetal rat long bones into the culture medium. The inhibitory effect of the compounds of formula (I) against bovine PTH induced bone resorption was expressed as mean percent inhibition ±

sem.

$^{45}Ca$-prelabelled fetal rat long bones (from forearms) were dissected and cultured in Linbro dishes at 37°C overnight BGJb medium, supplemented with lmg/ml BSA. There were five pairs of bones in each group. The compounds of formula (I) were dissolved in ethanol first, then diluted to various concentrations and added simultaneously with Bovine PTH (1-34) at $1x10^8$M on Day 1. The ethanol concentrations in the compound solutions were less than 0.05% which did not interfere with the assay. The assay was terminated on Day 6 with one media change on Day 3.

At the end of each medium change, the $^{45}Ca$ present in the culture medium was counted. The remaining bones were digested with 0.1N HCl and the $^{45}Ca$ presented in the bone digest was also counted. The results are expressed as % of the total $^{45}Ca$ released from each pair of bones. Bovine PTH at $1x10^{-8}$M induces bone resorption to the maximum level which is set as 100% and this concentration was used as standard. The level of base line bone resorption in the presence of medium only was set as 0%. All compound-treated groups were compared with bovine PTH (1-34) at $1x10^{-8}$M. The concentration at which a compound inhibited bone resorption by 50% was defined as $IC_{50}$.

The compounds of formula (Ia) exhibited the ability to inhibit bovine PTH-induced bone resorption in this assay.

| | $IC_{50}$ (molar) |
|---|---|
| Compound 1 | $\geq 5\times10^{-6}$ |
| Compound 2 | $2.7\times10^{-7}$ |
| Compound 3 | $5\times10^{-8}$ |
| Compound 4 | $\geq 5\times10^{-6}$ |

**TOXICOLOGY**

No serious toxicological effects were observed in the above described assays.

**Claims**

**1.** A compound of formula (I) :

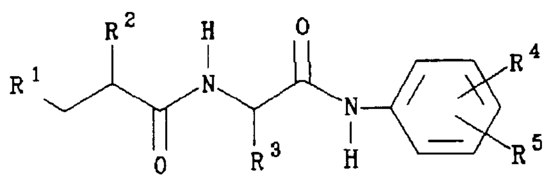

( I )

wherein

R¹ is mercapto, acetylthio, carboxy, hydroxycarbamoyl, alkoxycarbonyl, aryloxycarbonyl, aralkoxycarbonyl, benzyloxycarbamoyl or

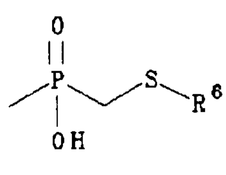

(where $R^6$ is optionally substituted aryl, wherein the aryl group is quinol-2-yl, naphth-1-yl, naphth-2-yl, pyridyl or phenyl);

$R^2$ is alkyl, aralkyl or cycloalkylalkyl;

$R^3$ is cycloalkyl, alkyl (optionally substituted by cycloalkyl, hydroxy, mercapto, alkylthio, aralkoxy, carboxy, amino, alkylamino, guanidino, carbamoyl, pyridyl or indolyl), or aralkyl (optionally substituted by hydroxy, carboxy, alkyl or alkoxy) ;

$R^4$ is nitro, amino, cyano, hydroxy, alkoxy, carboxy, alkoxycarbonyl, alkylsulfonyl, haloalkyl, alkoxycarbonylalkyl, tetrazolyl, carbamoyl (optionally substituted by alkyl or dialkylaminoalkyl) or aminosulfonyl (optionally substituted by alkyl); and

$R^5$ is hydrogen, halo or hydroxy; as a single stereoisomer or as a mixture thereof; or a pharmaceutically acceptable salt thereof.

**2.** A compound of Claim 1 wherein $R^1$ is mercapto or acetylthio.

**3.** The compound of Claim 2 wherein:

$R^3$ is cycloalkyl or alkyl (optionally substituted by cycloalkyl, hydroxy, aralkoxy, alkylthio, pyridyl or indolyl);
$R^4$ is cyano, hydroxy, alkoxy, carboxy, alkoxycarbonyl, alkoxycarbonylalkyl, carbamoyl (optionally substituted by aralkylaminoalkyl), or aminosulfonyl (optionally substituted by alkyl); and
$R^5$ is hydrogen.

**4.** A compound of Claim 3 wherein:

$R^2$ is alkyl;
$R^3$ is cyclohexyl or alkyl (optionally substituted by cyclohexyl, hydroxy, benzyloxy, methylthio, pyridyl or indolyl); and
$R^4$ is carboxy, alkoxycarbonyl, or aminosulfonyl.

**5.** A compound of Claim 4 wherein $R^2$ and $R^3$ are 2-methylpropyl.

**6.** A compound of Claim 5 wherein $R^1$ is mercapto or acetylthio and $R^4$ is methoxycarbonyl.

**7.** A single stereoisomer of the compounds of Claim 6, namely

*N*-(4-methyl-2-mercaptomethylpentanoyl)-L-leucine-*N'*-(4-methoxycarbonyl-phenyl)carboxamide; or *N*-(4-methyl-2-acetylthiomethylpentanoyl)-L-leucine-*N'*-(4-methoxycarbonyl-phenyl)carboxamide; or a pharmaceutically acceptable salt thereof.

**8.** A compound of Claim 1 wherein $R^1$ is carboxy or hydroxycarbamoyl.

**9.** A compound of Claim 8 wherein:

$R^3$ is cycloalkyl or alkyl (optionally substituted by cycloalkyl, hydroxy, aralkoxy, alkylthio, pyridyl or indolyl);
$R^4$ is cyano, hydroxy, alkoxy, carboxy, alkoxycarbonyl, alkoxycarbonylalkyl, carbamoyl (optionally substituted by aralkylaminoalkyl), or aminosulfonyl (optionally substituted by alkyl); and
$R^5$ is hydrogen.

**10.** A compound of Claim 9 wherein:

$R^2$ is alkyl;
$R^3$ is cyclohexyl or alkyl (optionally substituted by cyclohexyl, hydroxy, benzyloxy, methylthio, pyridyl or indolyl); and
$R^4$ is carboxy, alkoxycarbonyl, or aminosulfonyl.

**11.** A compound of Claim 10 wherein $R^2$ is 2-methylpropyl.

**12.** A compound of Claim 11 wherein $R^3$ is cyclohexyl, 2-methylpropyl, pyrid-3-ylmethyl, 1-benzyloxyethyl, 1-methylpropyl, 1,1-dimethylethyl, 1-hydroxyethyl, and indol-2-ylmethyl.

**13.** A compound of Claim 12 wherein $R^3$ is 2-methylpropyl and $R^4$ is carboxy or methoxycarbonyl.

**14.** A single stereoisomer of the compounds of Claim 13, namely

*N*-(4-methyl-2-carboxymethylpentanoyl)-L-leucine-*N'*-(4-methoxycarbonylphenyl)carboxamide;

*N*-(4-methyl-2-(*N*″-hydroxycarbamoyl)methylpentanoyl)-L-leucine-*N*′-(4-methoxycarbonylphenyl)carboxamide;
*N*-(4-methyl-2-(*N*″-hydroxycarbamoyl)methylpentanoyl)-L-leucine-*N*′-(4-carboxyphenyl)carboxamide;
*N*-(4-methyl-2-(*N*″-hydroxycarbamoyl)methylpentanoyl)-L-tryptophan-*N*′-(4-carboxyphenyl)carboxamide;
*N*-(4-methyl-2-(*N*″-hydroxycarbamoyl)methylpentanoyl)-L-cyclohexylglycine-*N*′-(4-methoxycarbonylphenyl) carboxamide; or
*N*-(4-methyl-2-(*N*″-hydroxycarbamoyl)methylpentanoyl)-L-*t*-leucine-*N*′-(4-methoxycarbonylphenyl)carboxamide; or a pharmaceutically acceptable salt thereof.

**15.** A pharmaceutical composition comprising a therapeutically effective amount of a compound of Claim 1, or a pharmaceutically acceptable salt thereof, in admixture with one or more pharmaceutically acceptable excipients.

**16.** Use of a compound represented by the formula

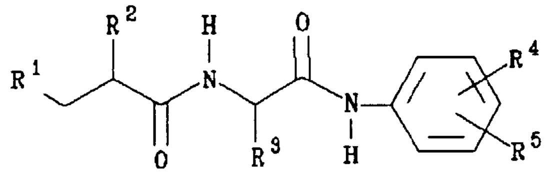

(I)

wherein

$R^1$ is mercapto, acetylthio, carboxy, hydroxycarbamoyl, alkoxycarbonyl, aryloxycarbonyl, aralkoxycarbonyl, benzyloxycarbamoyl or

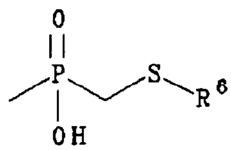

(where $R^6$ is optionally substituted aryl, wherein the aryl group is quinol-2-yl, naphth-1-yl, naphth-2-yl, pyridyl or phenyl);
$R^2$ is alkyl, aralkyl or cycloalkylalkyl;
$R^3$ is cycloalkyl, alkyl (optionally substituted by cycloalkyl, hydroxy, mercapto, alkylthio, aralkoxy, carboxy, amino, alkylamino, guanidino, carbamoyl, pyridyl or indolyl), or aralkyl (optionally substituted by hydroxy, carboxy, alkyl or alkoxy);
$R^4$ is nitro, amino, cyano, hydroxy, alkoxy, carboxy, alkoxycarbonyl, alkylsulfonyl, haloalkyl, alkoxycarbonylalkyl, tetrazolyl, carbamoyl (optionally substituted by alkyl or dialkylaminoalkyl), or aminosulfonyl (optionally substituted by alkyl); and
$R^5$ is hydrogen, halo or hydroxy; as a single stereoisomer or as a mixture thereof; or a pharmaceutically acceptable salt thereof,

in the manufacture of a medicament for inhibiting matrix metalloprotease activity in a mammal.

## Patentansprüche

**1.** Verbindung der Formel (I):

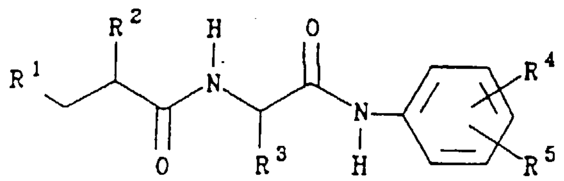

( I )

worin

$R^1$ Mercapto, Acetylthio, Carboxy, Hydroxycarbamoyl, Alkoxycarbonyl, Aryloxycarbonyl, Aralkoxycarbonyl, Benzyloxycarbamoyl oder

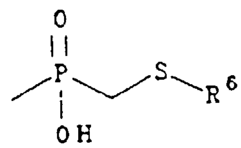

(worin $R^6$ für gegebenenfalls substituiertes Aryl steht, wobei die Arylgruppe Chinol-2-yl, Naphth-1-yl, Naphth-2-yl, Pyridyl oder Phenyl ist) ist;
$R^2$ Alkyl, Aralkyl oder Cycloalkylalkyl ist;
$R^3$ Cycloalkyl, Alkyl (gegebenenfalls substituiert durch Cycloalkyl, Hydroxy, Mercapto, Alkylthio, Aralkoxy, Carboxy, Amino, Alkylamino, Guanidino, Carbamoyl, Pyridyl oder Indolyl) oder Aralkyl (gegebenenfalls substituiert durch Hydroxy, Carboxy, Alkyl oder Alkoxy) ist;
$R^4$ Nitro, Amino, Cyano, Hydroxy, Alkoxy, Carboxy, Alkoxycarbonyl, Alkylsulfonyl, Halogenalkyl, Alkoxycarbonylalkyl, Tetrazolyl, Carbamoyl (gegebenenfalls substituiert durch Alkyl oder Dialkylaminoalkyl) oder Aminosulfonyl (gegebenenfalls substituiert durch Alkyl) ist; und
$R^5$ Wasserstoff, Halogen oder Hydroxy ist;

als einzelnes Stereoisomer oder als Mischung davon; oder ein pharmazeutisch annehmbares Salz derselben.

**2.** Verbindung nach Anspruch 1, in welcher $R^1$ Mercapto oder Acetylthio ist.

**3.** Verbindung nach Anspruch 2, in welcher:

$R^3$ Cycloalkyl oder Alkyl (gegebenenfalls substituiert durch Cycloalkyl, Hydroxy, Aralkoxy, Alkylthio, Pyridyl oder Indolyl) ist;
$R^4$ Cyano, Hydroxy, Alkoxy, Carboxy, Alkoxycarbonyl, Alkoxycarbonylalkyl, Carbamoyl (gegebenenfalls substituiert durch Aralkylaminoalkyl) oder Aminosulfonyl (gegebenenfalls substituiert durch Alkyl) ist; und
$R^5$ Wasserstoff ist.

**4.** Verbindung nach Anspruch 3, in welcher:

$R^2$ Alkyl ist;
$R^3$ Cyclohexyl oder Alkyl (gegebenenfalls substituiert durch Cyclohexyl, Hydroxy, Benzyloxy, Methylthio, Pyridyl oder Indolyl) ist; und
$R^4$ Carboxy, Alkoxycarbonyl oder Aminosulfonyl ist.

**5.** Verbindung nach Anspruch 4, in welcher $R^2$ und $R^3$ 2-Methylpropyl sind.

**6.** Verbindung nach Anspruch 5, in welcher $R^1$ Mercapto oder Acetylthio ist und $R^4$ Methoxycarbonyl ist.

**7.** Einzelnes Stereoisomer der Verbindungen nach Anspruch 6, nämlich

N-(4-Methyl-2-mercaptomethylpentanoyl)-L-leucin-N'-(4-methoxycarbonylphenyl)carboxamid; oder
N-(4-Methyl-2-acetylthiomethylpentanoyl)-L-leucin-N'-(4-methoxycarbonylphenyl)carboxamid; oder ein pharmazeutisch annehmbares Salz desselben.

**8.** Verbindung nach Anspruch 1, in welcher $R^1$ Carboxy oder Hydroxycarbamoyl ist.

**9.** Verbindung nach Anspruch 8, in welcher:

$R^3$ Cycloalkyl oder Alkyl (gegebenenfalls substituiert durch Cycloalkyl, Hydroxy, Aralkoxy, Alkylthio, Pyridyl oder Indolyl) ist;
$R^4$ Cyano, Hydroxy, Alkoxy, Carboxy, Alkoxycarbonyl, Alkoxycarbonylalkyl, Carbamoyl (gegebenenfalls substituiert durch Aralkylaminoalkyl) oder Aminosulfonyl (gegebenenfalls substituiert durch Alkyl) ist; und
$R^5$ Wasserstoff ist.

**10.** Verbindung nach Anspruch 9, in welcher

$R^2$ Alkyl ist;
$R^3$ Cyclohexyl oder Alkyl (gegebenenfalls substituiert durch Cyclohexyl, Hydroxy, Benzyloxy, Methylthio, Pyridyl oder Indolyl) ist; und
$R^4$ Carboxy, Alkoxycarbonyl oder Aminosulfonyl ist.

**11.** Verbindung nach Anspruch 10, in welcher $R^2$ 2-Methylpropyl ist.

**12.** Verbindung nach Anspruch 11, in welcher $R^3$ Cyclohexyi, 2-Methylpropyl, Pyrid-3-ylmethyl, 1-Benzyloxyethyl, 1-Methylpropyl, 1,1-Dimethylethyl, 1-Hydroxyethyl und Indol-2-ylmethyl ist.

**13.** Verbindung nach Anspruch 12, in welcher $R^3$ 2-Methylpropyl ist und $R^4$ Carboxy oder Methoxycarbonyl ist.

**14.** Einzelnes Stereoisomer der Verbindungen nach Anspruch 13, nämlich

N-(4-Methyl-2-carboxymethylpentanoyl)-L-leucin-*N'*-(4-methoxycarbonylphenyl)carboxamid;
N-(4-Methyl-2-(N"-hydroxycarbamoyl)methylpentanoyl)-L-leucin-N'-(4-methoxycarbonylphenyl)carboxamid;
N-(4-Methyl-2-(N'-hydroxycarbamoyl)methylpentanoyl)-L-leucin-N'-(4-carboxyphenyl)carboxamid;
N-(4-Methyl-2-(N"-hydroxycarbamoyl)methylpentanoyl)-L-tryptophan-N'-(4-carboxyphenyl)carboxamid;
N-(4-Methyl-2-(N"-hydroxycarbamoyl)methylpentanoyl)-L-cyclohexylglycin-N'-(4-methoxycarbonylphenyl) carboxamid; oder
N-(4-Methyl-2-(N"-hydroxycarbamoyl)methylpentanoyl)-L-t-leucin-N'-(4-methoxycarbonylphenyl)carboxamid; oder ein pharmazeutisch annehmbares Salz desselben.

**15.** Pharmazeutische Zusammensetzung, umfassend eine therapeutisch wirksame Menge einer Verbindung nach Anspruch 1 oder eines pharmazeutisch annehmbaren Salzes derselben in Mischung mit einem oder mehreren pharmazeutisch annehmbaren Exzipienten.

**16.** Verwendung einer Verbindung, dargestellt durch die Formel

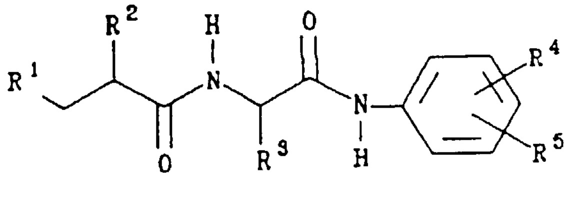

(I)

worin

$R^1$ Mercapto, Acetylthio, Carboxy, Hydroxycarbamoyl, Alkoxycarbonyl, Aryloxycarbonyl, Aralkoxycarbonyl, Benzyloxycarbamoyl oder

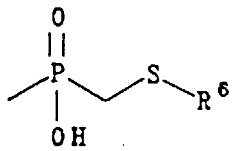

(worin $R^6$ für gegebenenfalls substituiertes Aryl steht, wobei die Arylgruppe Chinol-2-yl, Naphth-1-yl, Naphth-2-yl, Pyridyl oder Phenyl ist) ist;

$R^2$ Alkyl, Aralkyl oder Cycloalkylalkyl ist;

$R^3$ Cycloalkyl, Alkyl (gegebenenfalls substituiert durch Cycloalkyl, Hydroxy, Mercapto, Alkylthio, Aralkoxy, Carboxy, Amino, Alkylamino, Guanidino, Carbamoyl, Pyridyl oder Indolyl) oder Aralkyl (gegebenenfalls substituiert durch Hydroxy, Carboxy, Alkyl oder Alkoxy) ist;

$R^4$ Nitro, Amino, Cyano, Hydroxy, Alkoxy, Carboxy, Alkoxycarbonyl, Alkylsulfonyl, Halogenalkyl, Alkoxycarbonylalkyl, Tetrazolyl, Carbamoyl (gegebenenfalls substituiert durch Alkyl oder Dialkylaminoalkyl) oder Aminosulfonyl (gegebenenfalls substituiert durch Alkyl) ist; und

$R^5$ Wasserstoff, Halogen oder Hydroxy ist; als einzelnes Stereoisomer oder als Mischung davon oder eines pharmazeutisch annehmbaren Salzes derselben

bei der Herstellung eines Medikaments für die Inhibierung von Matrix-Metalloprotease-Aktivität in einem Säuger.

## Revendications

1. Composé de formule (I) :

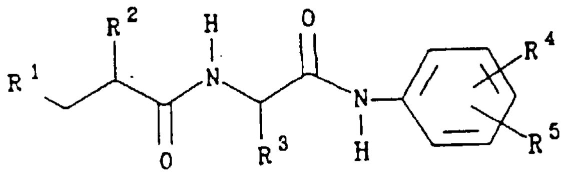

(I)

dans laquelle

$R^1$ représente un groupe mercapto, acétylthio, carboxy, hydroxycarbamoyle, alkoxycarbonyle, aryloxycarbonyle, aralkoxycarbonyle, benzyloxycarbamoyle ou

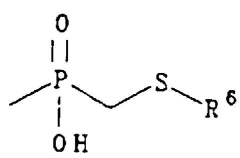

(dans laquelle $R^6$ représente un groupe aryle facultativement substitué, ledit groupe aryle consistant en un groupe quinole-2-yle, napht-1-yle, napht-2-yle, pyridyle ou phényle ;

$R^2$ représente un groupe alkyle, aralkyle ou cycloalkylalkyle ;

$R^3$ représente un groupe cycloalkyle, alkyle (facultativement substitué avec un substituant cycloalkyle, hydroxy, mercapto, alkylthio, aralkoxy, carboxy, amino, alkylamino, guanidino, carbamoyle, pyridyle ou indolyle), ou aralkyle (facultativement substitué avec un substituant hydroxy, carboxy, alkyle ou alkoxy) ;

$R^4$ représente un groupe nitro, amino, cyano, hydroxy, alkoxy, carboxy, alkoxycarbonyle, alkylsulfonyle, halogénalkyle, alkoxycarbonylalkyle, tétrazolyle, carbamoyle (facultativement substitué avec un substituant alkyle ou dialkylaminoalkyle) ou aminosulfonyle (facultativement substitué avec un substituant alkyle) ; et

$R^5$ représente l'hydrogène, un groupe halogéno, hydroxy ; sous forme d'un seul stéréo-isomère ou bien sous forme d'un mélange de stéréo-isomères de ce composés ; ou un de ses sels pharmaceutiquement acceptables.

**2.** Composé suivant la revendication 1, dans lequel $R^1$ représente un groupe mercapto ou acétylthio.

**3.** Composé suivant la revendication 2, dans lequel :

$R^3$ représente un groupe cycloalkyle ou alkyle (facultativement substitué avec un substituant cycloalkyle, hydroxy, aralkoxy, alkylthio, pyridyle ou indolyle) ;
$R^4$ représente un groupe cyano, hydroxy, alkoxy, carboxy, alkoxycarbonyle, alkoxycarbonylalkyle, carbamoyle (facultativement substitué avec un substituant aralkylaminoalkyle) ou aminosulfonyle (facultativement substitué avec un substituant alkyle) ; et
$R^5$ représente l'hydrogène.

**4.** Composé suivant la revendication 3, dans lequel :

$R^2$ représente un groupe alkyle ;
$R^3$ représente un groupe cyclohexyle ou alkyle (facultativement substitué avec un substituant cyclohexyle, hydroxy, benzyloxy, méthylthio, pyridyle ou indolyle) ; et
$R^4$ représente un groupe carboxy, alkoxycarbonyle ou aminosulfonyle.

**5.** Composé suivant la revendication 4, dans lequel $R^2$ et $R^3$ représentent des groupes 2-méthylpropyle.

**6.** Composé suivant la revendication 5, dans lequel $R^1$ représente un groupe mercapto ou acétylthio et $R^4$ représente un groupe méthoxycarbonyle.

**7.** Stéréo-isomère unique de composés suivant la revendication 6, à savoir

le N-(4-méthyl-2-mercaptométhylpentanoyl)-L-leucine-N'-(4-méthoxycarbonylphényl)carboxamide ; ou
le N-(4-méthyl-2-acétylthiométhylpentanoyl)-L-leucine-N'-(4-méthoxycarbonylphényl)carboxamide; ou un de ses sels pharmaceutiquement acceptables.

**8.** Composé suivant la revendication 1, dans lequel $R^1$ représente un groupe carboxy ou hydroxycarbamoyle.

**9.** Composé suivant la revendication 8, dans lequel :

$R^3$ représente un groupe cycloalkyle ou alkyle (facultativement substitué avec un substituant cycloalkyle, hydroxy, aralkoxy, alkylthio, pyridyle ou indolyle) ;
$R^4$ représente un groupe cyano, hydroxy, alkoxy, carboxy, alkoxycarbonyle, alkoxycarbonylalkyle, carbamoyle (facultativement substitué avec un substituant aralkylaminoalkyle) ou aminosulfonyle (facultativement substitué avec un substituant alkyle) ; et
$R^5$ représente l'hydrogène.

**10.** Composé suivant la revendication 9, dans lequel :

$R^2$ représente un groupe alkyle ;
$R^3$ représente un groupe cyclohexyle ou alkyle (facultativement susbstitué avec un substituant cyclohexyle, hydroxy, benzyloxy, méthylthio, pyridyle ou indolyle) ; et
$R^4$ représente un groupe carboxy, alkoxycarbonyle, ou aminosulfonyle.

**11.** Composé suivant la revendication 10, dans lequel $R^2$ représente un groupe 2-méthylpropyle.

**12.** Composé suivant la revendication 11, dans lequel $R^3$ représente un groupe cyclohexyle, 2-méthylpropyle, pyrid-3-ylméthyle, 1-benzyloxyéthyle, 1-méthylpropyle, 1,1-diméthyléthyle, 1-hydroxyéthyle ou indole-2-ylméthyle.

**13.** Composé suivant la revendication 12, dans lequel $R^3$ représente un groupe 2-méthylpropyle et $R^4$ représente un groupe carboxy ou méthoxycarbonyle.

**14.** Stéréo-isomère unique de composés suivant la revendication 13, à savoir

le N-(4-méthyl-2-carboxyméthylpentanoyl)-L-leucine-N'-(4-méthoxycarbonylphényl)carboxamide ;
le N-(4-méthyl-2-(N"-hydroxycarbamoyl)-méthylpentanoyl)-L-leucine-N'-(4-méthoxycarbonylphényl)-carboxamide ;
le N-(4-méthyl-2-(N"-hydroxycarbamoyl)méthylpentanoyl)-L-leucine-N'-(4-carboxyphényl)carboxamide ;
le N-(4-méthyl-2-(N"-hydroxycarbamoyl)méthylpentanoyl)-L-tryptophane-N'-(4-carboxyphényl)carboxamide ;
le N-(4-méthyl-2-(N"-hydroxycarbamoyl)méthylpentanoyl)-L-cyclohexylglycine--N'-(4-méthoxycarbonylphényl)carboxamide ; ou
le N-(4-méthyl-2-(N"-hydroxycarbamoyl)méthylpentanoyl)-L-t-leucine-N'-(4-méthoxycarbonylphényl)-carboxamide ;
ou un de ses sels pharmaceutiquement acceptables.

**15.** Composition pharmaceutique comprenant une quantité thérapeutiquement efficace d'un composé suivant la revendication 1, ou d'un de ses sels pharmaceutiquement acceptables, en mélange avec un ou plusieurs excipients pharmaceutiquement acceptables.

**16.** Utilisation d'un composé représenté par la formule

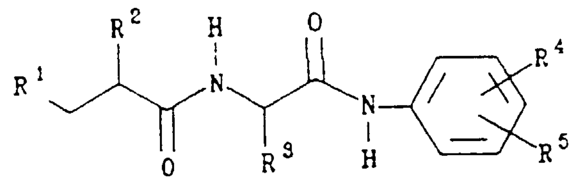

(I)

dans laquelle

$R^1$ représente un groupe mercapto, acétylthio, carboxy, hydroxycarbamoyle, alkoxycarbonyle, aryloxycarbonyle, aralkoxycarbonyle, benzyloxycarbamoyle ou

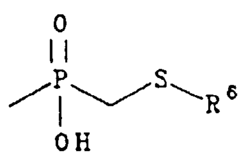

(dans lequel $R^6$ représente un groupe aryle facultativement substitué, le groupe aryle étant un groupe quinole-2-yle, napth-1-yle, napht-2-yle, pyridyle ou phényle) ;

$R^2$ représente un groupe alkyle, aralkyle ou cycloalkylalkyle ;

$R^3$ représente un groupe cycloalkyle, alkyle (facultativement substitué avec un substituant cycloalkyle, hydroxy, mercapto, alkylthio, aralkoxy, carboxy, amino, alkylamino, guanidino, carbamoyle, pyridyle ou indolyle) ou aralkyle (facultativement substitué avec un substituant hydroxy, carboxy, alkyle ou alkoxy) ;

$R^4$ représente un groupe nitro, amino, cyano, hydroxy, alkoxy, carboxy, alkoxycarbonyle, alkylsulfonyle, halogénalkyle, alkoxy-carbonylalkyle, tétrazolyle, carbamoyle (facultativement substitué avec un substituant alkyle ou dialkylaminoalkyle) ou aminosulfonyle (facultativement substitué avec un substituant alkyle) ;

$R^5$ représente l'hydrogène, un groupe halogéno ou hydroxy ; sous forme d'un seul stéréo-isomère ou bien sous forme d'un mélange de ces stéréo-isomères, ou un de ses sels pharmaceutiquement acceptables,

dans la production d'un médicament destiné à inhiber l'activité de métalloprotéase de matrice chez un mammifère.